# EUROPEAN PATENT APPLICATION

(11) **EP 4 067 356 A1**
(43) Date of publication of application: **05.10.2022**
(21) Application number: 20892878.8
(22) Date of filing: 27.11.2020
(51) Int. Cl.: C07D 471/14, A61K 31/437, A61P 19/02, A61P 29/00, A61P 37/00, A61P 35/00

(54) **TRI-HETEROCYCLIC COMPOUND AS JAK INHIBITOR, AND USE THEREOF**

(30) Priority: 27.11.2019 CN 201911180309; 04.06.2020 CN 202010501267
(71) Applicant: CHIA TAI TIANQING PHARMACEUTICAL GROUP CO., LTD., Lianyungang, Jiangsu 222062 (CN)
(72) Inventor: MAO, Weiwei, Shanghai 200131 (CN); LI, Wenlong, Shanghai 200131 (CN); WEI, Changqing, Shanghai 200131 (CN); QIAN, Wenyuan, Shanghai 200131 (CN); HU, Guoping, Shanghai 200131 (CN); LI, Jian, Shanghai 200131 (CN); CHEN, Shuhui, Shanghai 200131 (CN)
(74) Representative: v. Bezold & Partner Patentanwälte - PartG mbB
(86) International application number: PCT/CN2020/132426
(87) International publication number: WO 2021/104488

(57) **Abstract**

Disclosed are a tri-heterocyclic compound as a JAK inhibitor, and the use thereof in the preparation of a drug for treating JAK1- or/and JAK2-related diseases. Specifically, the present invention relates to a compound as shown in formula (I') or a pharmaceutically acceptable salt thereof.

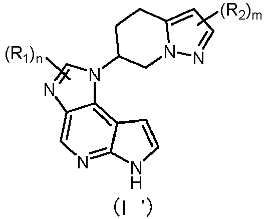

Sequence Listing

<110>  CHIA TAI TIANQING PHARMACEUTICAL GROUP CO., LTD.
       MEDSHINE DISCOVERY INC.

<120>  TRI-HETEROCYCLIC COMPOUND AS JAK INHIBITOR, AND USE THEREOF

<130>  PCTCN2020132426

<150>  201911180309.2
<151>  2019-11-27

<150>  202010501267.4
<151>  2020-06-04

<160>  4

<170>  PatentIn version 3.5

<210>  1
<211>  15
<212>  PRT
<213>  Artificial Sequence

<220>
<223>  peptide used in reaction of JAK1(h) enzyme

<400>  1

Met Gly Glu Glu Pro Leu Tyr Trp Ser Phe Pro Ala Lys Lys Lys
1               5                   10                  15


<210>  2
<211>  39
<212>  PRT
<213>  Artificial Sequence

<220>
<223>  peptide used in reaction of JAK2(h) enzyme

<400>  2

Lys Thr Phe Cys Gly Thr Pro Glu Tyr Leu Ala Pro Glu Val Arg Arg
1               5                   10                  15


Glu Pro Arg Ile Leu Ser Glu Glu Glu Gln Glu Met Phe Arg Asp Phe
            20                  25                  30


Asp Tyr Ile Ala Asp Trp Cys
        35


<210>  3
<211>  15
<212>  PRT
<213>  Artificial Sequence

<220>
<223>  peptide used in reaction of JAK3(h) enzyme

<400>  3

Gly Gly Glu Glu Glu Glu Tyr Phe Glu Leu Val Lys Lys Lys Lys
1               5                   10                  15


<210>  4
<211>  16
<212>  PRT
<213>  Artificial Sequence

<220>
<223>  peptide used in reaction of TYK2(h) enzyme

<400>  4

Gly Gly Met Glu Asp Ile Tyr Phe Glu Phe Met Gly Gly Lys Lys Lys
1               5                   10                  15

## Description

### CROSS-REFERENCE TO RELATED APPLICATIONS

The present application claims priority to and benefit of the Chinese Patent Application No. 201911180309.2 filed with China National Intellectual Property Administration on Nov. 27, 2019 and the Chinese Patent Application No. 202010501267.4 filed with China National Intellectual Property Administration on Jun. 4, 2020, the disclosures of which are incorporated herein by reference in their entirety.

### TECHNICAL FIELD

The present application relates to a tri-heterocyclic compound as a JAK inhibitor and use thereof in preparing a medicament for treating a JAK1- or/and JAK2-associated disease. Particularly, the present application relates to a compound of formula (I') or a pharmaceutically acceptable salt thereof.

### BACKGROUND

Janus kinases (JAKs) are a kind of cytoplasmic tyrosine kinase that can transmit cytokine signals from membrane receptors to STAT transcription factors. The JAK family comprises four members: JAK1, JAK2, JAK3, and TYK2. The JAK-STAT pathway transmits extracellular signals from a variety of cytokines, growth factors and hormones to the nucleus and is responsible for the expression of thousands of protein-coding genes. JAK-STAT intracellular signal transduction serves interferons, most interleukins, and a variety of cytokines and endocrine factors, such as EPO, TPO, GH, OSM, LIF, CNTF, GM-CSF, and PRL (Vainchenker W. et al. (2008)).

JAK-1, JAK-2 and TYK-2 are expressed in various tissue cells of the human body. JAK-3 is mainly expressed in various hematopoietic tissue cells and mainly exists in bone marrow cells, thymocytes, NK cells, and activated B lymphocytes and T lymphocytes. JAK1 has become a novel target in the disease fields of immunity, inflammation and cancer and the like. A base mutation JAK2V617F in the JAK2 gene in the human body is closely related to the occurrence of polycythemia vera (PV), essential thrombocythemia (ET), idiopathic myelofibrosis (IMF), chronic myeloid leukemia (CML), etc. in myeloproliferative diseases. Either JAK3 or γc mutations can cause severe combined immunodeficiency. Abnormal JAK3 activity manifests as a massive decrease in the number of T cells and NK cells, loss of the B cell function, or severely affecting normal biological functions of the immune system and the like. Based on its functional characteristics and special distribution in tissues, JAK3 has become an extremely attractive drug target for immune system-associated diseases. In mice, the loss of TYK2 function will cause the occurrence of defects in the signaling pathways of a variety of cytokine receptors, thereby leading to viral infections, decreased antibacterial immunity and increased probability of a pulmonary infection, etc. (John J.O'Shea, 2004, Nature Reviews Drug Discovery 3, 555-564). Different JAK family members selectively bind onto different cytokine receptors, giving signaling specificity to exert different physiological effects. Such selective action enables a JAK inhibitor to be relatively specifically applied to treat diseases. For example, the IL-2 or IL-4 receptor, along with the common γ chain, binds to JAK1 and JAK3, while type I receptors having the same β chain bind to JAK2. Type I receptors using gp130 (glycoprotein 130) and type I receptors activated by heterodimeric cytokines preferentially bind to JAK1/2 and TYK2. Type I receptors activated by hormone-like cytokines bind to and activate JAK2 kinase. Type II receptors for interferon bind to JAK1 and TYK2, while receptors for the IL-10 cytokine family bind to JAK1/2 and TYK2. The various kinds of specific binding of the cytokines and receptors thereof to the JAK family members described above trigger different physiological effects, making it possible to treat different diseases. JAK1 is heterodimerized with other JAKs to transduce cytokine-driven pro-inflammatory signaling. Therefore, inhibition of JAK1 and/or other JAKs is expected to be of therapeutic benefit for a range of inflammatory conditions and other diseases driven by JAK-mediated signal transduction (Daniella M. Schwartz, 2017, Nature Reviews Drug Discovery 16, 843-862).

### SUMMARY

In one aspect, the present application provides a compound of formula (I') or a pharmaceutically acceptable salt thereof, 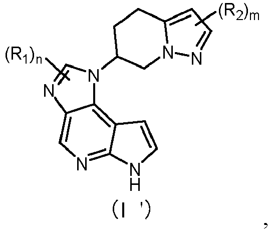 wherein,
m is 1, 2, 3, 4 or 5;
n is 1, 2, 3 or 4;
each R₁ is independently H, F, Cl, Br, I, CN, C₁₋₈ alkyl, C₁₋₈ alkoxy, C₁₋₈ alkyl S-, NH₂, C₁₋₈ alkyl NH-, (C₁₋₈ alkyl)₂N-, -COOH, or -C(O)OC₁₋₈ alkyl, wherein the C₁₋₈ alkyl, C₁₋₈ alkoxy, C₁₋₈ alkyl S-, C₁₋₈ alkyl NH-, (C₁₋₈ alkyl)₂N- or -C(O)OC₁₋₈ alkyl is each independently optionally substituted with 1, 2, 3 or 4 Rₐ;
each R₂ is independently H, F, Cl, Br, I, CN, NH₂, C₁₋₈ alkyl, C₁₋₈ alkoxy, C₁₋₈ alkyl S-, C₁₋₈ alkyl NH-, (C₁₋₈ alkyl)₂N-, or C₃₋₁₂ cycloalkyl;
each Rₐ is independently H, F, Cl, Br, I, OH, CN or NH₂.

In some embodiments of the present application, m is 1, 2 or 3. In some embodiments, m is 1 or 2. In some embodiments, m is 1.

In some embodiments of the present application, n is 1, 2 or 3. In some embodiments, n is 1 or 2. In some embodiments, n is 1.

In some embodiments of the present application, m and n are both 1.

In some embodiments of the present application, the structural fragment 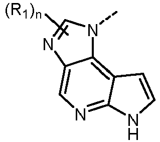 is 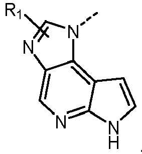 . In some embodiments, the structural fragment above is

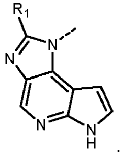

In some embodiments of the present application, the structural fragment 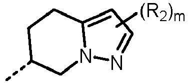 is 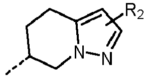 . In some embodiments, the structural fragment above is

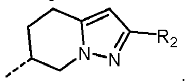

In some embodiments of the present application, each R₁ above is independently H, F, Cl, Br, I, CN, C₁₋₆ alkyl, C₁₋₆ alkoxy, C₁₋₆ alkyl S-, NH₂, C₁₋₆ alkyl NH-, (C₁₋₆ alkyl)₂N-, -COOH, or -C(O)OC₁₋₆ alkyl, wherein the C₁₋₆ alkyl, C₁₋₆ alkoxy, C₁₋₆ alkyl S-, C₁₋₆ alkyl NH-, (C₁₋₆ alkyl)₂N- or -C(O)OC₁₋₆ alkyl is each independently optionally substituted with 1, 2, 3 or 4 Rₐ.

In some embodiments of the present application, each R₁ above is independently H, F, Cl, Br, I, CN, C₁₋₆ alkyl, C₁₋₆ alkoxy, C₁₋₆ alkyl S-, NH₂, C₁₋₆ alkyl NH-, or (C₁₋₆ alkyl)₂N-, wherein the C₁₋₆ alkyl, C₁₋₆ alkoxy, C₁₋₆ alkyl S-, C₁₋₆ alkyl NH- or (C₁₋₆ alkyl)₂N- is each independently optionally substituted with 1, 2, 3 or 4 Rₐ.

In some embodiments of the present application, each R₁ above is independently H, F, Cl, Br, I, CN, C₁₋₃ alkyl, C₁₋₃ alkoxy, C₁₋₃ alkyl S-, NH₂, C₁₋₃ alkyl NH-, (C₁₋₃ alkyl)₂N-, -COOH, or -C(O)OC₁₋₃ alkyl, wherein the C₁₋₃ alkyl, C₁₋₃ alkoxy, C₁₋₃ alkyl S-, C₁₋₃ alkyl NH-, (C₁₋₃ alkyl)₂N- or -C(O)OC₁₋₃ alkyl is each independently optionally substituted with 1, 2, 3 or 4 Rₐ.

In some embodiments of the present application, each R₁ above is independently H, F, Cl, Br, I, CN, C₁₋₃ alkyl, C₁₋₃ alkoxy, C₁₋₃ alkyl S-, NH₂, C₁₋₃ alkyl NH-, or (C₁₋₃ alkyl)₂N-, wherein the C₁₋₃ alkyl, C₁₋₃ alkoxy, C₁₋₃ alkyl S-, C₁₋₃ alkyl NH- or (C₁₋₃ alkyl)₂N- is each independently optionally substituted with 1, 2, 3 or 4 Rₐ.

In some embodiments of the present application, each R₁ above is independently H, F, Cl, Br, I, CN, C₁₋₃ alkyl, C₁₋₃ alkoxy, C₁₋₃ alkyl S-, or C₁₋₃ alkyl NH-, wherein the C₁₋₃ alkyl, C₁₋₃ alkoxy, C₁₋₃ alkyl S- or C₁₋₃ alkyl NH- is each independently optionally substituted with 1, 2, 3 or 4 Rₐ.

In some embodiments of the present application, each R₁ above is independently H, F, Cl, Br, I, CN, C₁₋₃ alkyl or C₁₋₃ alkoxy, wherein the C₁₋₃ alkyl or C₁₋₃ alkoxy is each independently optionally substituted with 1, 2, 3 or 4 Rₐ.

In some embodiments of the present application, each R₂ above is independently H, F, Cl, Br, I, CN, NH₂, C₁₋₆ alkyl, C₁₋₆ alkoxy, C₁₋₆ alkyl S-, C₁₋₆ alkyl NH-, (C₁₋₆ alkyl)₂N-, or C₃₋₁₀ cycloalkyl.

In some embodiments of the present application, each R₂ above is independently H, F, Cl, Br, I, CN, NH₂, C₁₋₃ alkyl, C₁₋₃ alkoxy, C₁₋₃ alkyl S-, C₁₋₃ alkyl NH-, (C₁₋₃ alkyl)₂N-, or C₃₋₆ cycloalkyl.

In some embodiments of the present application, each R₂ above is independently H, F, Cl, Br, I, CN, NH₂, C₁₋₃ alkyl, C₁₋₃ alkoxy, C₁₋₃ alkyl S-, or C₁₋₃ alkyl NH-.

In some embodiments, each R₂ above is independently H, F, Cl, Br, I, CN or NH₂.

In some embodiments of the present application, each R₂ above is independently H, F, Cl, Br, I or CN.

In some embodiments of the present application, each Rₐ above is independently F, Cl, Br, I or OH.

In some embodiments of the present application, the compound of formula (I') or the pharmaceutically acceptable salt thereof described above is a compound of formula (I) or a pharmaceutically acceptable salt thereof, 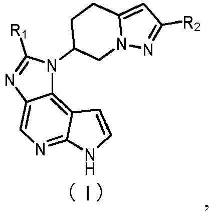 wherein,
R₁ is H, F, Cl, Br, I, CN, C₁₋₃ alkyl or C₁₋₃ alkoxy, wherein the C₁₋₃ alkyl and C₁₋₃ alkoxy are each independently optionally substituted with 1, 2, 3 or 4 Rₐ;
R₂ is H, F, Cl, Br, I or CN;
each Rₐ is independently H, F, Cl, Br, I or OH.

In some embodiments of the present application, each R₁ above is independently H, F, Cl, Br, I, CN, -CH₃, -CH₂CH₃, or -OCH₃, wherein the -CH₃, -CH₂CH₃ and -OCH₃ are each independently optionally substituted with 1, 2, 3 or 4 Rₐ, and the other variables are as defined in the present application.

In some embodiments of the present application, each R₁ above is independently H, F, Cl, Br, I, CN, -CH₃, -CH₂CH₃ or -OCH₃, wherein the -CH₃, -CH₂CH₃ and -OCH₃ are each independently optionally substituted with 1, 2 or 3 Rₐ, and the other variables are as defined in the present application.

In some embodiments of the present application, each R₁ above is independently H, F, Cl, Br, I, CN, -CH₃, -CH₂CH₃, or -OCH₃, wherein the -CH₃ or -CH₂CH₃ is each independently optionally substituted with 1, 2 or 3 Rₐ, and the other variables are as defined in the present application.

In some embodiments of the present application, each Rₐ above is independently F, Cl, Br, I, or OH, and the other variables are as defined in the present application.

In some embodiments of the present application, each Rₐ above is independently F or OH, and the other variables are as defined in the present application.

In some embodiments of the present application, each R₁ above is independently H, F, Cl, Br, I, CN, -CH₃, -CH₂CH₃, or -OCH₃, wherein the -CH₃ or -CH₂CH₃ is each independently optionally substituted with 1, 2 or 3 F or OH, and the other variables are as defined in the present application.

In some embodiments of the present application, each R₁ above is independently H, F, Cl, Br, I, CN, -CH₃, -CF₃, -CH₂CH₃, -CH(OH)CH₃, or -OCH₃, and the other variables are as defined in the present application.

In some embodiments of the present application, each R₁ above is independently H, CN, -CH₃, -CF₃, -CH(OH)CH₃, or -OCH₃, and the other variables are as defined in the present application.

In some embodiments of the present application, each R₁ above is independently H, F, Cl, Br, I, CN, -CH₃, -CF₃, -CH₂CH₃, or -OCH₃, and the other variables are as defined in the present application.

In some embodiments of the present application, each R₁ above is independently H, CN, -CH₃, -CF₃, -CH₂CH₃, or -OCH₃, and the other variables are as defined in the present application.

In some embodiments of the present application, each R₂ above is independently F, Cl, Br, I, or CN, and the other variables are as defined in the present application.

In some embodiments of the present application, each R₂ above is independently CN, and the other variables are as defined in the present application.

Some other embodiments of the present application are derived from any combination of the variables described above.

In some embodiments of the present application, the compound of formula (I') or the pharmaceutically acceptable salt thereof described above is a compound of formula (I'-A) or formula (I'-B) or a pharmaceutically acceptable salt thereof: 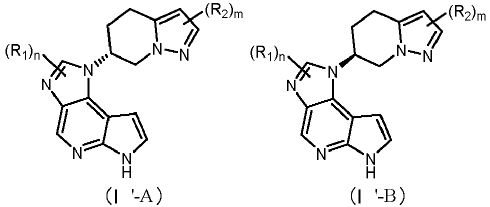 wherein R₁, R₂, m or n is defined as in the present application.

In some embodiments of the present application, the compound of formula (I) or the pharmaceutically acceptable salt thereof described above is a compound of formula (I-A) or formula (I-B) or a pharmaceutically acceptable salt thereof: 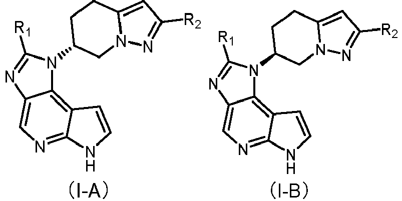 wherein R₁ or R₂ is defined as in the present application.

In some embodiments of the present application, the compound of formula (I') or formula (I) or the pharmaceutically acceptable salt thereof described above has a structure of formula (I-1): 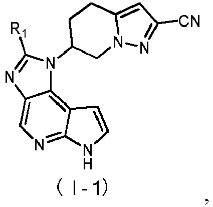 wherein,
R₁ is as defined in the present application.

In some embodiments of the present application, the compound of formula (1-1) or the pharmaceutically acceptable salt thereof described above is a compound of formula (I-1-A) or formula (I-1-B) or a pharmaceutically acceptable salt thereof: 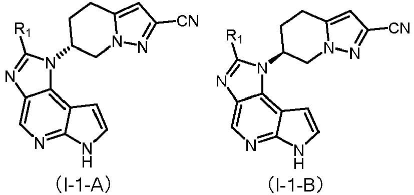 wherein R₁ is as defined in the present application.

In another aspect, the present application also provides a compound of the formula below or a pharmaceutically acceptable salt thereof,

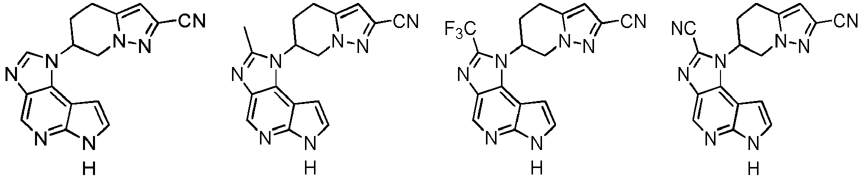

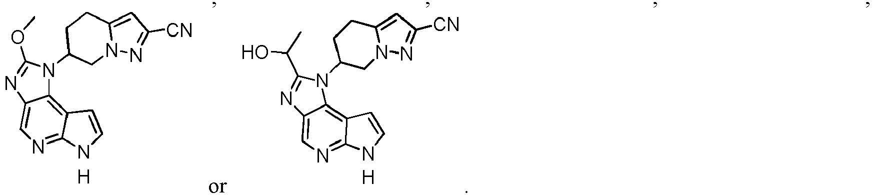

In some embodiments of the present application, the compound or the pharmaceutically acceptable salt thereof described above is selected from: 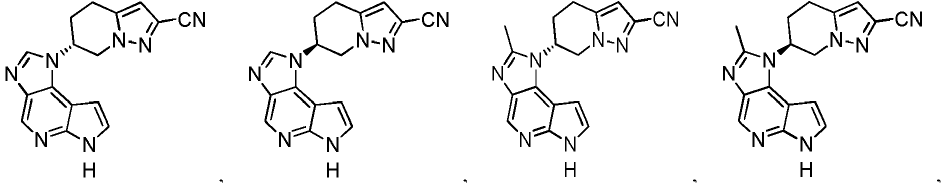 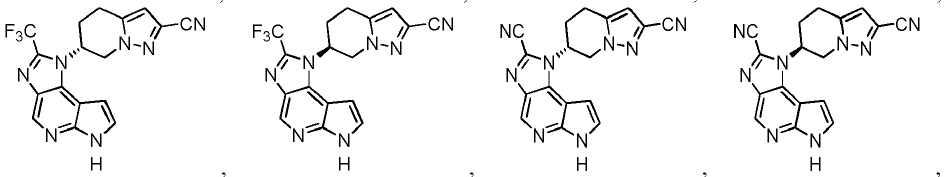 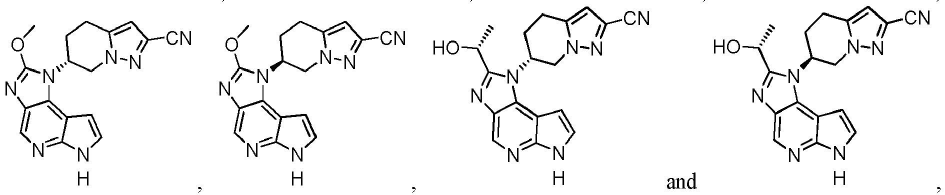 or a pharmaceutically acceptable salt thereof.

In yet another aspect, the present application also provides a pharmaceutical composition comprising the compound or the pharmaceutically acceptable salt thereof of the present application described above. In some embodiments, the pharmaceutical composition of the present application further comprises a pharmaceutically acceptable excipient.

In yet another aspect, the present application also provides use of the compound or the pharmaceutically acceptable salt thereof described above in the preparation of a drug for treating a JAK1- or/and JAK2-associated disease.

In yet another aspect, the present application also provides use of the compound or the pharmaceutically acceptable salt thereof described above for treating a JAK1- or/and JAK2-associated disease.

In yet another aspect, the present application also provides the compound or the pharmaceutically acceptable salt thereof for use in treating a JAK1- or/and JAK2-associated disease.

In yet another aspect, the present application also provides a method for treating a JAK1- or/and JAK2-associated disease comprising administering to a mammal, preferably a human, in need thereof a therapeutically effective amount of the compound or the pharmaceutically acceptable salt thereof or the pharmaceutical composition thereof described above.

In the present application, the JAK1- or/and JAK2-associated disease is selected from inflammatory conditions (e.g., arthritis) and the like.

### Technical Effects

The compound of the present application exhibits good selective inhibition of JAK1 and/or JAK2 in *in vitro* activity assays of the 4 subtypes of the kinase, JAK1, JAK2, JAk3 and TYK2; and has good oral bioavailability and high exposure in murine, favoring the generation of good *in vivo* efficacy.

### Definitions and Description

Unless otherwise stated, the following terms and phrases used herein are intended to have the following meanings. A particular term or phrase should not be considered uncertain or unclear in the absence of a specific definition, but should be construed according to its common meaning. When referring to a trade name herein, it is intended to refer to its corresponding commercial product or its active ingredient.

The dotted line ( ---- ) in a structural unit or group in the present application represents a covalent bond.

When a bond of a substituent is cross-linked to two atoms on a ring, the substituent can be bonded to any atom on the ring. For example, the structural unit 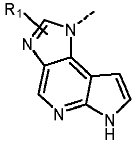 indicates that it may be substituted at any one position of all rings, including particularly but not limited to

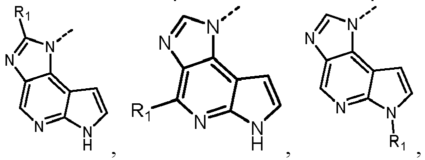

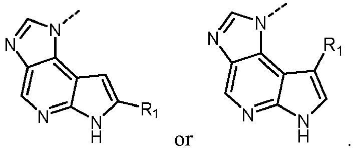

The term "pharmaceutically acceptable" is used herein for those compounds, materials, compositions and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound of the present application, which is prepared from the compound having particular substituents discovered by the present application and a relatively nontoxic acid or base. When the compound of the present application contains a relatively acidic functional group, a base addition salt can be obtained by contacting such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound of the present application contains a relatively basic functional group, an acid addition salt can be obtained by contacting such a compound with a sufficient amount of an acid in a pure solution or a suitable inert solvent. Examples of pharmaceutically acceptable acid addition salts include salts of inorganic acids, which comprise such as hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid and phosphorous acid; and salts of organic acids, which comprise such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid and methanesulfonic acid. Also included are salts of amino acids (e.g., arginine) and salts of organic acids such as glucuronic acid. Certain specific compounds of the present application contain both basic and acidic functional groups that allow the compounds to be converted into any base or acid addition salts.

The pharmaceutically acceptable salts of the present application can be synthesized from a parent compound having an acidic or basic group through conventional chemical methods. In general, such salts are prepared by the following method: reacting the free acid or base form of the compound with a stoichiometric amount of the appropriate base or acid in water or an organic solvent or a mixture thereof.

The compounds of the present application can occur in the form of a specific geometric isomer or stereoisomer. All such compounds are contemplated herein, including *cis* and *trans* isomers, (-)- and (+)-enantiomers, (*R*)- and (*S*)- enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present application. Substituents such as alkyl may have an additional asymmetric carbon atom. All these isomers and mixtures thereof are encompassed within the scope of the present application. For example, it will be understood by those skilled in the art that the compound of formula (I') of the present application includes, but is not limited to, the compound of formula (I'-A) or formula (I'-B) and a mixture thereof formed in any ratio, that the compound of formula (I) of the present application includes, but is not limited to, the compound of formula (I-A) or formula (I-B) and a mixture thereof formed in any ratio, and that the compound of formula (1-1) of the present application includes, but is not limited to, the compound of formula (I-1-A) or formula (I-1-B) and a mixture thereof formed in any ratio, and the like.

Unless otherwise stated, the term "enantiomer" or "optical isomer" refers to stereoisomers that are mirror images of each other.

Unless otherwise stated, the term *"cis-trans* isomer" or "geometric isomer" results from the inability of a single bond of a ring carbon atom or a double bond to rotate freely.

Unless otherwise stated, the term "diastereoisomer" refers to stereoisomers whose molecules have two or more chiral centers and are not mirror images of each other.

Unless otherwise stated, "(+)" stands for dextrorotation, "(-)" stands for levorotation, and "(±)" stands for racemization.

Unless otherwise stated, the absolute configuration of a stereogenic center is represented by a wedged solid bond ( 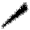 ) and a wedged dashed bond ( 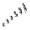 ), and the relative configuration of a stereogenic center is represented by a straight solid bond ( 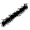 ) and a straight dashed bond ( 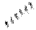 ). A wavy line ( 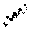 ) represents a wedged solid bond ( 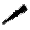 ) or a wedged dashed bond ( 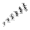 ), or a wavy line ( 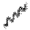 ) represents a straight solid bond ( 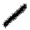 ) and a straight dashed bond ( 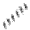 ).

Unless otherwise stated, when a double bond structure such as a carbon-carbon double bond, a carbon-nitrogen double bond, and a nitrogen-nitrogen double bond is present in a compound, and each atom on the double bond is linked to two different substituents (in the double bond including nitrogen atom(s), a lone pair of electrons on the nitrogen atom(s) is regarded as a substituent to which the nitrogen atom is linked), if the atom on the double bond of the compound and its substituents are linked using a wavy line ( 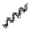 ), it means that the compound exists in the form of a (*Z*)-type isomer, a (*E*)-type isomer, or a mixture of the two isomers. For example, the following formula (A) represents that the compound exists in the form of a single isomer of formula (A-1) or formula (A-2) or in the form of a mixture of both isomers of formula (A-1) and formula (A-2); the following formula (B) represents that the compound exists in the form of a single isomer of formula (B-1) or formula (B-2) or in the form of a mixture of both isomers of formula (B-1) and formula (B-2); and the following formula (C) represents that the compound exists in the form of a single isomer of formula (C-1) or formula (C-2) or in the form of a mixture of both isomers of formula (C-1) and formula (C-2).

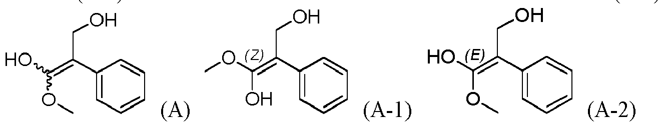

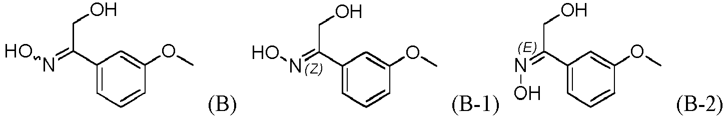

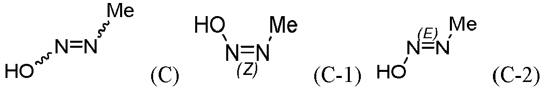

Unless otherwise stated, the term "tautomer" or "tautomeric form" means that different functional isomers are in dynamic equilibrium at room temperature and can be rapidly converted into each other. If tautomers are possible (e.g., in solution), the chemical equilibrium of the tautomers can be achieved. For example, a proton tautomer, also known as a prototropic tautomer, includes the interconversion by proton transfer, such as keto-enol isomerization and imine-enamine isomerization. A valence tautomer includes the interconversion by recombination of some bonding electrons. A specific example of the keto-enol tautomerization is the interconversion between the two tautomers of pentane-2,4-dione and 4-hydroxypent-3-en-2-one.

Unless otherwise stated, the term of "being enriched in one isomer", "isomer enrichment", "being enriched in one enantiomer", or "enantiomer enrichment" means that the content of one of the isomers or enantiomers is less than 100%, and more than or equal to 60%, 70%, 80%, 90%, 95%, 96%, 97%, 98%, 99%, 99.5%, 99.6%, 99.7%, 99.8% or 99.9%.

Unless otherwise stated, the term "isomeric excess" or "enantiomeric excess" refers to the difference between the relative percentages of two isomers or enantiomers. For example, if the content of one isomer or enantiomer is 90% and the content of the other isomer or enantiomer is 10%, the isomeric or enantiomeric excess (ee) is 80%.

Optically active (*R*)- and (*S*)-isomers and *D* and *L* isomers can be prepared by chiral synthesis or chiral reagents or other conventional techniques. If an enantiomer of a certain compound of the present application is desired, it can be prepared by asymmetric synthesis or derivatization using a chiral auxiliary, wherein the resulting diastereoisomeric mixture is separated and the auxiliary group is cleaved to provide the desired pure enantiomer. Alternatively, when a molecule contains a basic functional group (such as amino) or an acidic functional group (such as carboxyl), it reacts with an appropriate optically active acid or base to form a salt of the diastereoisomer, which is then subjected to diastereoisomeric resolution through conventional methods in the art to give the pure enantiomer. Furthermore, the enantiomer and the diastereoisomer are generally isolated through chromatography using a chiral stationary phase, optionally in combination with chemical derivatization (e.g., carbamate generated from amines).

The compound of the present application may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted with deuterium to form a deuterated drug, and the bond formed by deuterium and carbon is firmer than that formed by common hydrogen and carbon. Compared with an un-deuterated drug, the deuterated drug has the advantages of reduced toxic side effects, increased stability, enhanced efficacy, prolonged drug biological half-life and the like. All isotopic variations of the compound described herein, whether radioactive or not, are encompassed within the scope of the present application.

"Optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted with substituents which may include deuterium and hydrogen variants, as long as the valence of the specific atom is normal and the substituted compound is stable. When the substituent is oxo (i.e., =O), it means that two hydrogen atoms are substituted. Substitution with oxygen does not occur on aromatic groups. The term "optionally substituted" means that it can be substituted or unsubstituted. Unless otherwise specified, the type and number of the substituent may be arbitrary as long as being chemically achievable.

When any variable (e.g., R) occurs one or more times in the constitution or structure of a compound, the variable is independently defined in each case. Thus, for example, if a group is substituted with 0-2 R, the group can be optionally substituted with up to two R, and the definition of R in each case is independent. Furthermore, a combination of a substituent and/or a variant thereof is permissible only if the combination can result in a stable compound.

When the number of a linking group is 0, for example, -(CRR)₀- means that the linking group is a single bond. When one of variables is selected from a single bond, it means that the two groups linked by it are linked directly. For example, in A-L-Z, when L represents a single bond, it means that the structure is actually A-Z. When a substituent is absent, it means that there is no such a substituent. For example, when X is absent in A-X, it means that the structure is actually A. When it is not specified by which atom the listed substituent is linked to the group to be substituted, the substituent can be bonded via its any atom. For example, pyridinyl as a substituent can be linked to the group to be substituted via any carbon atom on the pyridine ring.

The term "alkyl" refers to hydrocarbyl with a general formula of CₙH₂ₙ₊₁. The alkyl can be linear or branched. For example, the term "C₁₋₆ alkyl" refers to alkyl containing 1 to 6 carbon atoms (for example, methyl, ethyl, *n*-propyl, isopropyl, *n*-butyl, isobutyl, *sec*-butyl, *tert-*butyl*, n*-pentyl, 1-methylbutyl, 2-methylbutyl, 3-methylbutyl, neopentyl, hexyl, 2-methylpentyl, *etc.*)*.* The alkyl moieties (namely alkyl) of alkoxy, alkylamino, dialkylamino, alkylsulfonyl and alkylthio are similarly defined as above.

Unless otherwise specified, the term "C₁₋₃ alkyl" refers to a linear or branched saturated hydrocarbon group consisting of 1 to 3 carbon atoms. The C₁₋₃ alkyl includes, but is not limited to, C₁₋₂ and C₂-₃ alkyl, etc., and may be monovalent (e.g., methyl), divalent (e.g., methylene) or polyvalent (e.g., methenyl). Examples of C₁₋₃ alkyl include, but are not limited to, methyl (Me), ethyl (Et), propyl (including *n*-propyl and isopropyl), etc. The term "alkoxy" refers to "alkyl-O-". Unless otherwise specified, the term "C₁₋₃ alkoxy" refers to those alkyl groups that each contains 1 to 3 carbon atoms and is linked to the rest part of the molecule via an oxygen atom. The C₁₋₃ alkoxy includes, but is not limited to, C₁₋₂, C₂₋₃, C₃ and C₂ alkoxy, and the like. Examples of C₁₋₃ alkoxy include, but are not limited to, methoxy, ethoxy, propoxy (including *n*-propoxy and isopropoxy), etc. Unless otherwise specified, C_{n to n+m} or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁ and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc. For example, "C₁₋₆" means that the group may contain 1 carbon atom, 2 carbon atoms, 3 carbon atoms, 4 carbon atoms, 5 carbon atoms or 6 carbon atoms. Similarly, n to n+m membered means that the ring contains n to n+m atoms. For example, 3-12 membered ring includes 3 membered ring, 4 membered ring, 5 membered ring, 6 membered ring, 7 membered ring, 8 membered ring, 9 membered ring, 10 membered ring, 11 membered ring and 12 membered ring. Also, any range within n to n+m may be included. For example, 3-12 membered ring includes 3-6 membered ring, 3-9 membered ring, 5-6 membered ring, 5-7 membered ring, 6-7 membered ring, 6-8 membered ring, 6-10 membered ring, etc.

The term "cycloalkyl" refers to a carbon ring that is fully saturated and may exist as a single ring, a bridged ring, or a spiro ring. Unless otherwise specified, the carbon ring is generally a 3-10 membered ring. Non-limiting examples of cycloalkyl groups include, but are not limited to, cyclopropyl, cyclobutyl, cyclopentyl, cyclohexyl, norbornyl (bicyclo[2.2.1]heptyl), bicyclo[2.2.2]octyl, adamantyl, etc.

The term "aryl" refers to an all-carbon monocyclic or fused polycyclic aromatic ring group with the conjugated pi-electron system. For example, an aryl may have 6-20 carbon atoms, 6-14 carbon atoms or 6-12 carbon atoms. Non-limiting examples of aryl include, but are not limited to, phenyl, naphthyl, anthryl, 1,2,3,4-tetrahydronaphthalenyl, etc.

The term "leaving group" refers to a functional group or atom replaced by another functional group or atom through a substitution reaction (e.g., nucleophilic substitution reaction). For example, representative leaving groups include triflate; chlorine, bromine and iodine; sulfonate groups, such as mesylate, tosylate, p-bromobenzenesulfonate, and p-toluenesulfonate; and acyloxy groups, such as acetoxy and trifluoroacetoxy. The term "protecting group" includes, but is not limited to," amino protecting group", "hydroxy protecting group" or "sulfydryl protecting group". The term "amino protecting group" refers to a protecting group suitable for preventing side reactions at the nitrogen atom of the amino. Representative amino protecting groups include, but are not limited to: formyl; acyl, such as alkanoyl (such as acetyl, trichloroacetyl or trifluoroacetyl); alkoxycarbonyl, such as *tert*-butoxycarbonyl (Boc); arylmethoxycarbonyl, such as carbobenzoxy (Cbz) and 9-fluorenylmethoxycarbonyl (Fmoc); arylmethyl, such as benzyl (Bn), trityl (Tr), 1,1-di-(4'-methoxyphenyl)methyl; and silyl, such as trimethylsilyl (TMS) and *tert*-butyldimethylsilyl (TBS). The term "hydroxyl protecting group" refers to a protecting group suitable for preventing side reactions of the hydroxyl group. Representative hydroxy protecting groups include, but are not limited to: alkyl, such as methyl, ethyl, and *tert-butyl*; acyl, such as alkanoyl (such as acetyl); arylmethyl, such as benzyl (Bn), *p*-methoxybenzyl (PMB), 9-fluorenylmethyl (Fm) and diphenylmethyl (benzhydryl, DPM); and silyl, such as trimethylsilyl (TMS) and *tert*-butyldimethylsilyl (TBS).

The term "treat" or "treatment" means administering the compound or formulation of the present application to ameliorate or eliminate a disease or one or more symptoms associated with the disease, and includes:
(i) inhibiting a disease or disease state, i.e., arresting its development; and
(ii) alleviating a disease or disease state, i.e., causing its regression.

The term "prevent" or "prevention" means administering the compound or formulation of the present application to prevent a disease or one or more symptoms associated with the disease, and includes: preventing the occurrence of the disease or disease state in a mammal, particularly when such a mammal is predisposed to the disease state but has not yet been diagnosed with it.

The word "comprise" and variations thereof such as "comprises" or "comprising" will be understood in an open, non-exclusive sense, i.e., "including but not limited to".

The compounds of the present application can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The term "pharmaceutical composition" refers to a mixture consisting of one or more of the compounds or the salts thereof of the present application and a pharmaceutically acceptable excipient. The pharmaceutical composition is intended to facilitate the administration of the compound to an organism.

The term "pharmaceutically acceptable excipient" refers to those which do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, water.

The pharmaceutical composition of the present application can be prepared by combining the compound of the present application with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous and intravenous administration.

The pharmaceutical composition of the present application can be manufactured using methods well known in the art, such as conventional mixing, dissolving, granulating, dragee-making, grinding, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compound of the present application to be formulated into tablets, pills, pastilles, dragees, capsules, liquids, gels, slurries, suspensions, etc. for oral administration to a patient.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners or flavoring agents and the like.

The pharmaceutical compositions may also be suitable for parenteral administration, such as sterile solutions, suspensions or lyophilized products in suitable unit dosage forms.

In all of the administration methods of the compound of the present application, the daily dose administered is from 0.01 to 200 mg/kg body weight, in a single or separated doses.

The compound of the present application may be structurally confirmed through conventional methods well known to those skilled in the art; if the present application relates to the absolute configuration of the compound, the absolute configuration may be confirmed by means of conventional techniques in the art. For example, in the single crystal X-ray diffraction (SXRD) method, intensity data of diffraction of the single crystal grown are collected with a Bruker D8 venture diffractometer, the light source is CuKα radiation, and the scanning mode is ϕ/ω scanning; after related data are collected, the direct method (Shelxs97) is further employed to analyze the crystal structure, and thus the absolute configuration can be confirmed.

In some embodiments, the compound of the present application can be prepared by those skilled in the art of organic synthesis according to the scheme below: 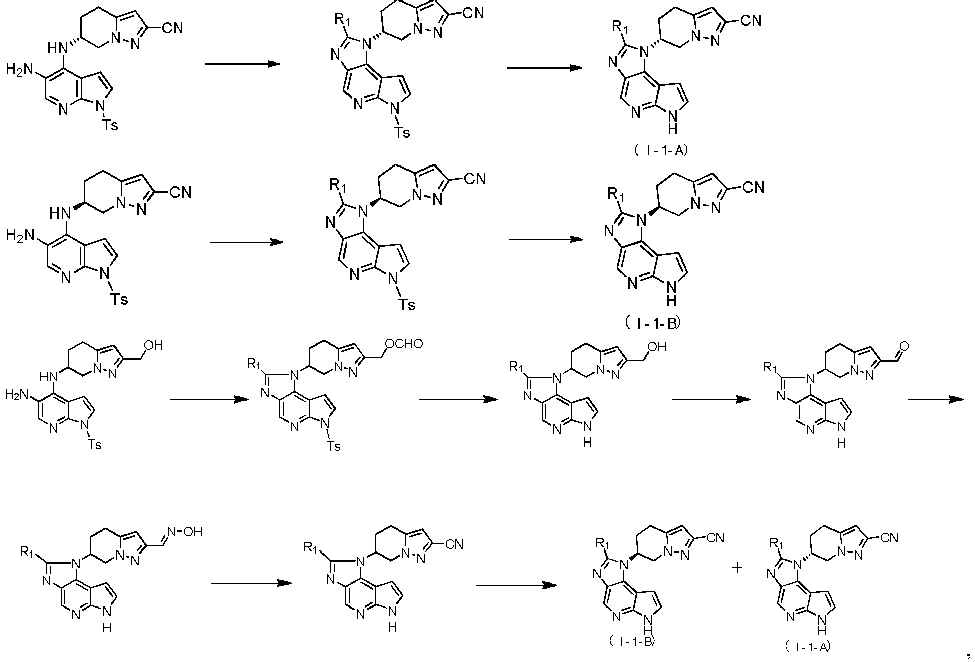 wherein R₁ is as defined in the present application.

The solvents used in the present application are commercially available. The present application uses the following abbreviations: aq for water; *eq* for equivalent; DCM for dichloromethane; PE for petroleum ether; EA for ethyl acetate; DMF for *N,N*-dimethylformamide; DMSO for dimethyl sulfoxide; EtOAc for ethyl acetate; EtOH for ethanol; MeOH for methanol; Boc for *tert*-butyloxycarbonyl, an amine protecting group; AcOH or HOAc for acetic acid; r.t. for room temperature; THF for tetrahydrofuran; TFA for trifluoroacetic acid; Tₛ for *p*-toluenesulfonyl; TBS for *tert*-butyldimethylsilyl; PMB for *p*-methoxybenzyl; HPMC for hydroxypropyl methylcellulose; PVP for polyvinylpyrrolidone; SLS for sodium lauryl sulfate; SBE-β-CD for sulfobutyl ether-P-cyclodextrin; TLC for thin layer chromatography; DMAP for 4-dimethylaminopyridine; DEA for diethylamine; ADDP for 1,1'-azodicarbonyldipiperidine; TBAF for tetrabutylammonium fluoride; TMSI for iodotrimethylsilane; DIEA for *N,N*-diisopropylethylamine; TsOH for *p*-toluenesulfonic acid; EDCI for l-ethyl-(3-dimethylaminopropyl)carbodiimide hydrochloride; HOBt for 1-hydroxybenzotriazole; BSA for bovine serum albumin; EDTA for ethylenediamine tetraacetic acid; and BID for twice a day.

### DETAILED DESCRIPTION

The present application is described in detail by way of examples. However, this is by no means disadvantageously limiting the scope of the present application. Although the present application has been described in detail herein and specific embodiments have also been disclosed, it will be apparent to those skilled in the art that various changes and modifications can be made to the specific embodiments without departing from the spirit and scope of the present application.

### Example 1: Synthesis of Compounds 1A and 1B

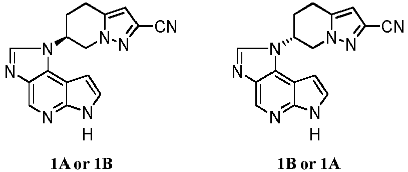

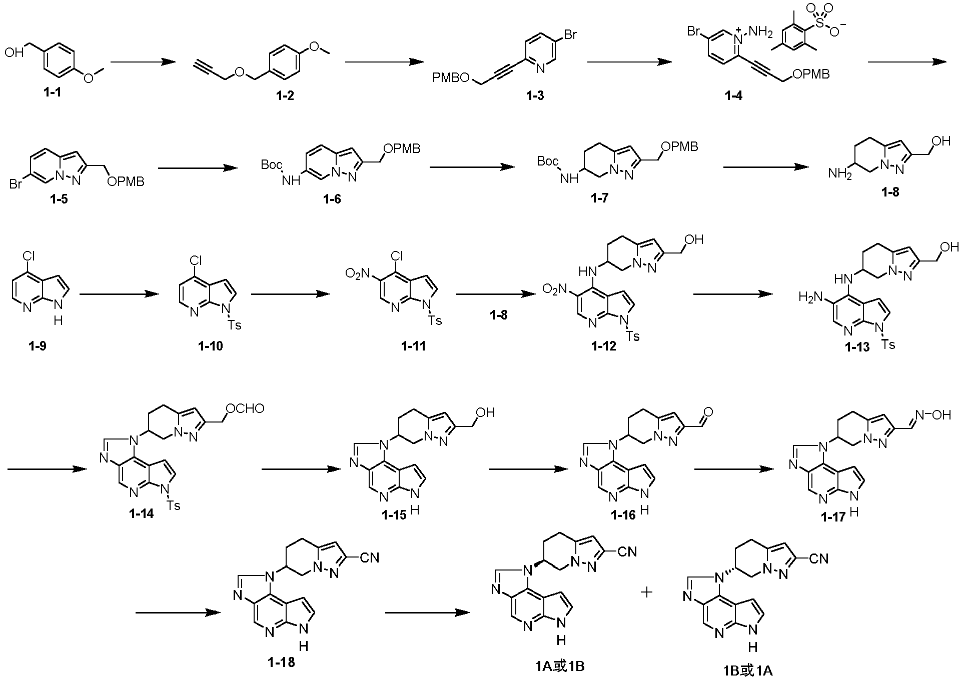

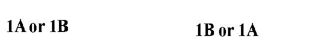

Step 1: (4-methoxyphenyl)methanol (50 g, 361.89 mmol, 45.05 mL, 1 *eq)* was added into a suspension of sodium hydride (15.92 g, 398.08 mmol, 60% purity, 1.1 *eq)* in DMF (200 mL) at 0 °C; after 0.5 h of stirring, 3-bromoprop-1-yne (59.19 g, 398.08 mmol, 42.89 mL, 1.1 *eq)* was added slowly into the reaction system; the resulting solution was stirred at 0 °C for 0.5 h and stirred at 25 °C for 16 h; the reaction was completed and a new major product was produced, as indicated by TLC (PE : EA = 10:1); saturated aqueous ammonium chloride solution (50 mL) was added into the reaction solution, and the aqueous phase was extracted with ethyl acetate (500 mL × 3); the organic phases were pooled, washed successively with water (200 mL × 2) and brine (200 mL × 1), dried over anhydrous sodium sulfate, and filtered; the filtrate was concentrated under reduced pressure, and the resulting residue was purified by column chromatography (SiO₂, PE : EA = 1:0 to 5:1) to give compound **1-2.** MS (ESI) calcd. for C_{II}H₁₂O₂: 176; found: 177 [M+H]⁺.

Step 2: compound 1-2 (34.14 g, 193.74 mmol, 25.00 mL, 1.1 *eq*)*,* copper(I) iodide (3.35 g, 17.59 mmol, 0.1 *eq*)*,* piperidine (44.99 g, 528.36 mmol, 52.18 mL, 3 *eq*) and dichlorobis(triphenylphosphine)palladium(II) (6.18 g, 8.80 mmol, 0.05 *eq*) were added into a solution of 5-bromo-2-iodo-pyridine (50 g, 176.12 mmol, 1 *eq*) in THF (500 mL) at 25 °C, and the reaction system was subjected to nitrogen purging three times; the resulting solution was stirred at 25 °C for 16 h; the reaction was completed and a new major product was produced, as indicated by TLC (PE : EA = 10:1); the reaction solution was filtered through diatomite, and the resulting filtrate was concentrated under reduced pressure; the residue was dissolved in 800 mL of ethyl acetate, and then washed successively with 300 mL of water and 300 mL of brine; the organic phase was dried over anhydrous sodium sulfate and filtered; the filtrate was concentrated under reduced pressure, and the resulting residue was subjected to column chromatography (SiO₂, PE : EA = 1:0 to 30:1) to give compound 1-3. ¹H NMR (400 MHz, CCl3-*d*) δ = 8.61 (d, *J* = 1.8 Hz, 1H), 7.75 (dd, *J* = 2.4, 8.4 Hz, 1H), 7.32 - 7.28 (m, 1H), 7.32 - 7.26 (m, 1H), 7.27 (s, 1H), 6.89 - 6.84 (m, 2H), 4.58 (s, 2H), 4.35 (s, 2H), 3.79 - 3.76 (m, 1H), 3.77 (s, 2H). MS (ESI) calcd. for C₁₆H₁₄BrNO₂: 332; found: 334. MS (ESI) calcd. for C₁₆H₁₄BrNO₂: 331, 333; found: 331 [M]⁺, 334 [M+H]⁺.

Step 3: ammonium 2,4,6-trimethylbenzenesulfonate (972 mg, 4.52 mmol, 1.5 *eq)* was dissolved in dry DCM (15 mL) containing anhydrous Na₂SO₄, and then compound **1-3** (1 g, 3.01 mmol, 1 *eq*) was added into the resulting mixture at 0 °C, which was then stirred at 25 °C for 16 h. A small amount of the reaction materials remained and a new major product was produced, as indicated by TLC (PE : EA = 2:1 ). 32 mL of *tert-*butyl methyl ether was slowly added into the system with stirring at 0 °C, and a large amount of off-white solid slowly precipitated; the solid was filtrated and dried to give compound **1-4.** MS (ESI) calcd. for C₁₆H₁₆BrN₂O₂: 348; found: 348 [M]⁺.

Step 4: silver carbonate (10.07 g, 36.52 mmol, 1.66 mL, 2 *eq)* was added into a solution of compound **1-4** (10 g, 18.27 mmol, 1 *eq)* in DMF (160 mL) at 25 °C, and the resulting solution was stirred at 40 °C for 16 h. The starting materials were completely reacted and a product was produced, as indicated by LCMS; the reaction solution was filtered through diatomite, and the resulting filtrate was concentrated under reduced pressure; the residue was dissolved in 200 mL of ethyl acetate, and the resulting solution was washed successively with 100 mL of water and 100 mL of brine; the organic phase was then dried over anhydrous sodium sulfate and filtered; the filtrate was concentrated under reduced pressure, and the resulting residue was subjected to column chromatography (SiO₂, PE : EA = 1:0 to 5:1) to give compound **1-5.** ¹H NMR (400 MHz, CCl₃-*d*) δ = 8.50 - 8.46 (m, 1H), 7.34 - 7.27 (m, 1H), 7.26 - 7.21 (m, 2H), 7.10 - 7.06 (m, 1H), 6.84 - 6.78 (m, 2H), 6.50 - 6.46 (m, 1H), 4.67 - 4.60 (m, 2H), 4.51 - 4.46 (m, 2H), 3.75 - 3.71 (m, 3H). MS (ESI) calcd. for C₁₆H₁₅BrN₂O₂: 347; found: 348.8 [M+H]⁺.

Step 5: potassium hydroxide (1.63 g, 29.05 mmol, 2.15 *eq),* 5-(di-*tert*-butylphosphino)-1,3,5-triphenyl-1*H*-[1,4|bipyrazole (783.26 mg, 1.55 mmol, 0.1 *eq*) and tris(dibenzylideneacetone)dipalladium(0) (619.79 mg, 676.83 µmol, 0.05 *eq*) were sequentially added into a mixed solution of compound **1-5** (4.7 g, 13.54 mmol, 1 *eq*) and *tert-*butyl carbamate (1.9 g, 16.22 mmol, 1.2 *eq*) in isoamyl alcohol (15 mL) and water (15 mL) at 25 °C, and the reaction system was subjected to nitrogen purging three times; the resulting solution was stirred at 100 °C for 2 h; the starting materials were completely reacted and a product was produced, as indicated by LCMS; the reaction solution was filtered through diatomite to obtain the filtrate, and the filter cake was washed with 100 mL of ethyl acetate; the organic phases were pooled, washed successively with 100 mL of water and 100 mL of brine, dried over anhydrous sodium sulfate, and filtered; the filtrate was concentrated under reduced pressure, and the resulting residue was purified by column chromatography (SiO₂, PE : EA = 1:0 to 20:1) to give compound **1-6.** MS (ESI) calcd. for C₂₁H₂₅N₃O₄: 383; found: 384 [M+H]⁺.

Step 6: platinum dioxide (933.33 mg, 4.11 mmol, 0.1 *eq*) was added to a solution of compound **1-6** (4 g, 40.43 mmol, 1 *eq*) in EtOH (60 mL) under nitrogen atmosphere at 25 °C, and the reaction system was subjected to H₂ purging three times; the resulting solution was stirred at 70 °C under H₂ (3 MPa) for 72 h; the starting materials were completely reacted and a product was produced, as indicated by LCMS; the reaction solution was filtered through diatomite, and the resulting filtrate was concentrated under reduced pressure to give compound **1-7.** MS (ESI) calcd. for C₂₁H₂₉N₃O₄: 387; found: 388 [M+H]⁺.

Step 7: trifluoroacetic acid (12.33 g, 108.14 mmol, 8.00 mL, 10.85 *eq*) was added into a solution of compound **1-7** (4 g, 10.32 mmol, 1 *eq*) in DCM (40 mL) at 0 °C, and the resulting solution was stirred at 25 °C for 2 h; the starting materials were completely reacted and a product was produced, as indicated by LCMS; the reaction mixture was concentrated under reduced pressure to give compound **1-8.** MS (ESI) calcd. for CsH₁₃N₃O: 167; found: 168 [M+H]⁺.

Step 8: *p*-toluenesulfonyl chloride (27.49 g, 144.19 mmol, 1.1 *eq*)*,* DMAP (1.6 g, 13.11 mmol, 0.1 *eq*) and triethylamine (19.9 g, 196,66 mmol, 27.37 mL) were added into a solution of compound **1-9** (20 g, 131.08 mmol, 1 *eq*) in DCM (50 mL) at 25 °C, respectively, and the resulting solution was stirred at 25 °C for 16 h; the starting materials were completely reacted as indicated by LCMS; the solvent was removed under reduced pressure, and a saturated solution of NaHCO3 (50 mL) was added thereto; the resulting mixture was filtered, and the filter cake was washed with water and dried to give compound **1-10**. MS (ESI) calcd. for C₁₄H₁₁N₂ClSO₂: 306; found: 307 [M+H]⁺.

Step 9: a solution of tetrabutylammonium nitrate (29.78 g, 97.81 mmol, 3 *eq*) in dichloromethane (50 mL) was added dropwise into a solution of compound **1-10** (10 g, 32.60 mmol, 1 *eq*) in DCM (50 mL) at -5 °C, and trifluoroacetic anhydride (20.54 g, 97.79 mmol, 13.60 mL, 3 *eq*) was slowly added dropwise; the resulting solution was stirred at -5 °C for 30 min, and then stirred at 25 °C for 16 h; the starting materials were completely reacted as indicated by LCMS; ethyl acetate (500 mL × 3) was used for extraction, and the organic phases were pooled, washed successively with water (200 mL × 2) and brine (200 mL × 1), dried over anhydrous sodium sulfate, and filtered; the filtrate was concentrated under reduced pressure, and the residue was recrystallized from dichloromethane to give product **1-11.** MS (ESI) calcd. for C₁₄H₁₀N₃ClSO₄: 351; found: 352 [M+H]⁺.

Step 10: *N,N*-diisopropyletlhylamine (15.43 g, 119.40 mmol, 20.8 mL, 10 *eq*) was added into a solution of compound **1-11** (4.2 g, 11.94 mmol, 1 *eq*) and compound **1-8** (3.69 g, 13.12 mmol, 1.1 *eq*) in isopropanol (50 mL) at 25 °C, and the resulting solution was stirred at 90 °C for 16 h; the starting materials were completely reacted and a product was produced, as indicated by LCMS; the reaction mixture was concentrated by rotary evaporation, and water (500 mL) was added; the aqueous phase was extracted with ethyl acetate (500 mL × 3), and the organic phases were pooled, washed successively with water (200 mL × 2) and brine (200 mL × 1), dried over anhydrous sodium sulfate, and filtered; the filtrate was concentrated under reduced pressure, and the resulting residue was purified by column chromatography (SiO₂, DCM : MeOH = 100:1 to 50:1) to give compound **1-12.** MS (ESI) calcd. for C₂₂H₂₂N₆O₅S: 482; found: 483 [M+H]⁺.

Step 11: palladium on carbon (0.1 g, 10%) was added into a solution of compound **1-12** in methanol (20 mL) at 25 °C under nitrogen, and the reaction system was subjected to hydrogen purging three times; the resulting solution was stirred at 25 °C for 16 h; the starting materials were completely reacted and a product was produced, as indicated by LCMS; the reaction solution was filtered through diatomite, and the resulting filtrate was concentrated under reduced pressure to give compound **1-13.** MS (ESI) calcd. for C₂₂H₂₄N₆O₃S: 452; found: 453 [M+H]⁺.

Step 12: compound **1-13** (2 g, 4.42 mmol, 1 *eq)* was dissolved in formic acid (10 mL), and the resulting solution was stirred at 110 °C for 16 h; the starting materials were completely reacted and a product was produced, as indicated by LCMS; the reaction solution was concentrated by rotary evaporation to give compound **1-14.** MS (ESI) calcd. for C₂₄H₂₂N₆O₄S: 490; found: 491 [M+H]⁺.

Step 13: 10% aqueous sodium hydroxide solution (10 mL) was added dropwise into a solution of compound **1-14** (1.50 g, 3.06 mmol, 1 *eq)* in methanol (10 mL) at 25 °C, and the resulting solution was stirred at 25 °C for 4 h; the starting materials were completely reacted and a product was produced, as indicated by LCMS; the reaction solution was neutralized with 10% diluted hydrochloric acid, and the aqueous phase was extracted with dichloromethane (100 mL × 3); the organic phases were pooled, washed with saturated brine (100 mL × 1), and concentrated by rotary evaporation to give compound **1-15.** MS (ESI) calcd. for C₁₆H₁₆N₆O: 308; found: 309 [M+H]⁺.

Step 14: manganese dioxide (2.83 g, 32.6 mmol, 10 *eq*) was added into a solution of compound **1-15** (1 g, 3.24 mmol, 1 *eq)* in a mixed solvent of DCM (20 mL) and MeOH (2 mL) at 25 °C, and the resulting solution was stirred at 65 °C for 16 h; the starting materials were completely reacted and a product was produced, as indicated by LCMS; the reaction solution was filtered through diatomite, and the resulting filtrate was concentrated under reduced pressure to give compound **1-16.** MS (ESI) calcd. for C₁₆H₁₄N₆O: 306; found: 307 [M+H]⁺.

Step 15: hydroxylamine hydrochloride (106.99 mg, 1.66 mmol, 1.2 *eq*) and sodium acetate (126.29 mg, 1.54 mmol, 1.2 *eq*) were added into a solution of compound **1-16** (0.39 g, 1.27 mmol, 1 *eq*) in methanol (15 mL) at 25 °C, and the resulting solution was stirred at 25 °C for 0.5 h; the starting materials were completely reacted and a product was produced, as indicated by LCMS; the reaction mixture was concentrated under reduced pressure, and the resulting residue was diluted with 25 mL of saturated brine, and extracted with THF (25 mL × 3); the organic phase was dried over anhydrous sodium sulfate and filtered, and the filtrate was concentrated under reduced pressure to give compound **1-17.** MS (ESI) calcd. for C₁₆H₁₅N₇O: 321; found: 322 [M+H]⁺. Step 16: thiocarbonyldiimidazole (388.2 mg, 2.18 mmol, 2 *eq*) was added into a solution of compound **1-17** (0.35 g, 1.09 mmol, 1 *eq*) in THF (15 mL) at 25 °C, and the resulting solution was stirred at 25 °C for 16 h; the starting materials were completely reacted and a product was produced, as indicated by LCMS; water (50 mL) was added to the reaction solution, and the aqueous phase was extracted with dichloromethane (100 mL × 3); the organic phases were pooled, dried over anhydrous sodium sulfate, and filtered; the filtrate was concentrated under reduced pressure, and the resulting residue was purified by column chromatography (SiO₂, DCM : MeOH = 50:0 to 5:1) to give compound **1-18.** ¹H NMR(400 MHz, DMSO-*d*₆) δ = 11.91 (s, 1H), 8.62 (s, 1H), 8.22 (s, 1H), 7.50 - 7.48 (s, 1H), 6.92 - 6.88 (m, 1H), 5.53 - 5.20 (s, 1H), 4.84 - 4.80 (m, 1H), 4.73 - 4.67 (m, 1H), 3.34 - 3.32 (m, 2H), 3.02 - 2.49 (m, 2H). MS (ESI) calcd. for C₁₆H₁₃N₇: 303; found: 304 [M+H]⁺.

Step 17: compound **1-18** was subjected to chiral resolution (chiral column: Chiralcel OJ-3 50 × 4.6 mm I.D., 3 µm ; mobile phases: supercritical CO₂ as phase A, and MeOH (0.05% DEA) as phase B; gradient: B content in A from 5% to 40%; flow rate: 3 mL/min; wavelength: 220 nm; column temperature: 35 °C; back pressure: 100 Bar) to give compound **1A** (retention time: 0.917 min) and compound **1B** (retention time: 2.790 min). Compound **1A**: ¹H NMR (400 MHz, DMSO-*d*₆) δ = 11.91 (s, 1H), 8.62 (s, 1H), 8.22 (s, 1H), 7.50 - 7.48 (s, 1H), 6.92 - 6.88 (m, 1H), 5.53 - 5.20 (s, 1H), 4.84 - 4.80 (m, 1H), 4.73 - 4.67 (m, 1H), 3.34 - 3.32 (m, 2H), 3.02 - 2.49 (m, 2H). C₁₆H₁₃N₇: 303; found: 304 [M + H]⁺.

### Example 2: Synthesis of Compounds 2A and 2B

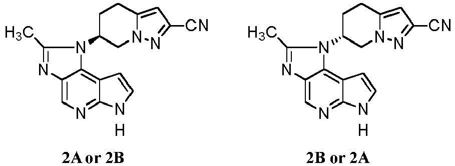

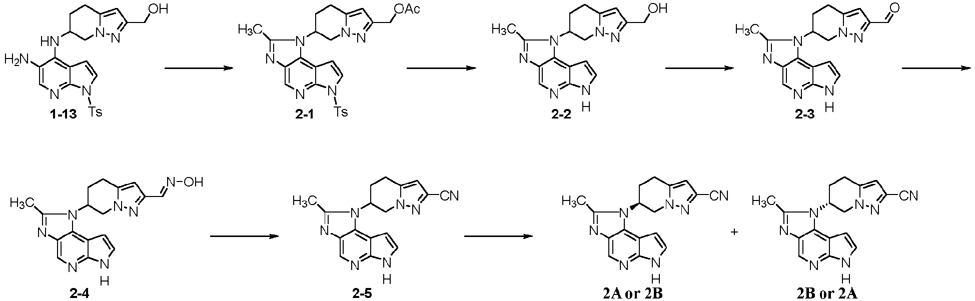

Step 1: compound 1-13 (2.0 g, 4.40 mmol, 1 *eq*) was dissolved in acetic acid (20 mL), and the resulting solution was stirred at 120 °C for 15 h; the starting materials were completely reacted and a product was produced, as indicated by LCMS; the reaction mixture was concentrated by rotary evaporation to give compound **2-1**. MS (ESI) calcd. for C₂₆H₂₆N₆O₄S: 518; found: 519 [M+H]⁺.

Step 2: 10% aqueous sodium hydroxide solution (10 mL) was added dropwise into a solution of compound **2-1** (1.50 g, 2.89 mmol, 1 *eq*) in methanol (10 mL) at 25 °C, and the resulting solution was stirred at 25 °C for 4 h; the starting materials were completely reacted and a product was produced, as indicated by LCMS; the reaction solution was neutralized with 10% diluted hydrochloric acid, and the aqueous phase was extracted with dichloromethane (100 mL × 3); the organic phases were pooled, washed with saturated brine (100 mL × 1), and concentrated by rotary evaporation to give compound **2-2.** MS (ESI) calcd. for C₁₇H₁₈N₆O: 322; found: 323 [M+H]⁺.

Step 3: manganese dioxide (2.7 g, 31.0 mmol, 10 *eq*) was added into a solution of compound **2-2** (1 g, 3.1 mmol, 1 *eq*) in a mixed solvent of DCM (20 mL) and MeOH (2 mL) at 25 °C, and the resulting solution was stirred at 65 °C for 16 h; the starting materials were completely reacted and a product was produced, as indicated by LCMS; the reaction solution was filtered through diatomite, and the resulting filtrate was concentrated under reduced pressure to give compound **2-3.** MS (ESI) calcd. for C₁₇H₁₆N₆O: 320; found: 321 [M+H]⁺.

Step 4: hydroxylamine hydrochloride (119.3 mg, 1.54 mmol, 1.1 *eq)* and sodium acetate (192.1 mg, 2.34 mmol, 1.5 *eq)* were added into a solution of compound **2-3** (0.5 g, 1.56 mmol, 1 *eq)* in methanol (50 mL) at 25 °C, and the resulting solution was stirred at 25 °C for 0.5 h; the starting materials were completely reacted and a product was produced, as indicated by LCMS; the reaction solution was concentrated under reduced pressure, and the resulting residue was diluted with 25 mL of saturated brine, and extracted with THF (25 mL × 3); the organic phase was dried over anhydrous sodium sulfate and filtered, and the filtrate was concentrated under reduced pressure to give compound **2-4.** MS (ESI) calcd. for C₁₇H₁₇N₇O: 335; found: 336 [M+H]⁺.

Step 5: thiocarbonyldiimidazole (371.9 mg, 2.09 mmol, 2 *eq*) was added into a solution of compound **2-4** (0.35 g, 1.04 mmol, 1 *eq*) in THF (15 mL) at 25 °C, and the resulting solution was stirred at 25 °C for 16 h; the starting materials were completely reacted and a product was produced, as indicated by LCMS; water (50 mL) was added into the reaction solution, and the aqueous phase was extracted with dichloromethane (100 mL × 3); the organic phases were pooled, dried over anhydrous sodium sulfate, and filtered; the filtrate was concentrated under reduced pressure, and the resulting residue was subjected to prep-HPLC (column: Phenomenex Gemini-NX 80 × 40 mm × 3 µm; mobile phase: [Water (0.05% NH₃H₂O + 10 mM NH₄HCO₃)-ACN]; ACN: 17%-37%, 8 min) to give compound **2-5**. ¹H NMR (400 MHz, DMSO-*d*₆) δ = 11.88 (s, 1H), 8.52 (s, 1H), 7.43 (s, 1H), 6.90 (s, 1H), 6.35 - 6.34 (m, 1H), 5.36 - 5.34 (m, 1H), 4.82 - 4.68 (m, 2H), 3.52 - 3.37 (m, 2H), 2.69 - 2.58 (m, 4H), 2.50 - 2.29 (m, 1H); MS (ESI) calcd. for C₁₇H₁₅N₇: 317; found: 318 [M+H]⁺.

Step 6: compound **2-5** was subjected to chiral resolution (chiral column: Chiralcel OJ-3 50 × 4.6 mm I.D., 3 µm ; mobile phases: supercritical CO₂ as phase A, and MEOH (0.05% DEA) as phase B; gradient: B content in A from 5% to 40%; flow rate: 3 mL/min; wavelength: 220 nm; column temperature: 35 °C; back pressure: 100 Bar) to give compound **2A** (retention time: 3.198 min) and compound **2B** (retention time: 3.947 min).

### Example 3: Synthesis of Compounds 3A and 3B

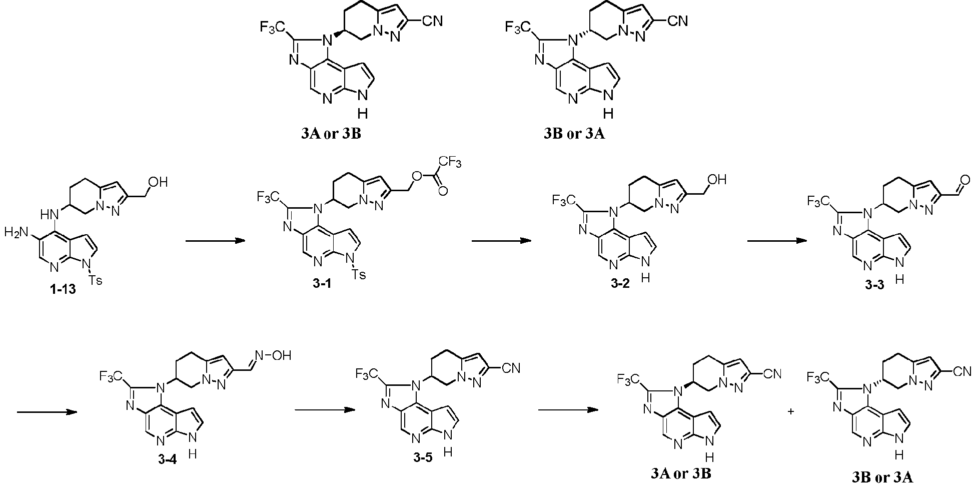

Step 1: compound **1-13** (2 g, 4.40 mmol, 1 *eq*) was dissolved in trifluoroacetic acid (10 mL), and the resulting solution was stirred at 110 °C for 16 h; the starting materials were completely reacted and a product was produced, as indicated by LCMS; the reaction mixture was concentrated by rotary evaporation to give compound **3-1**. MS (ESI) calcd. for C₂₆H₂₀F₆N₆O₄S: 626; found: 627 [M+H]⁺.

Step 2: 10% aqueous sodium hydroxide solution (10 mL) was added dropwise into a solution of compound 3-1 (1.50 g, 2.39 mmol, 1 *eq)* in methanol (10 mL) at 25 °C, and the resulting solution was stirred at 25 °C for 4 h; the starting materials were completely reacted and a product was produced, as indicated by LCMS; the reaction solution was neutralized with 10% diluted hydrochloric acid, and the aqueous phase was extracted with dichloromethane (100 mL × 3); the organic phases were pooled, washed with saturated brine (100 mL × 1), and concentrated by rotary evaporation to give compound **3-2.** MS (ESI) calcd. for C₁₇H₁₅F₃N₆O: 376; found: 377 [M+H]⁺.

Step 3: manganese dioxide (2.3 g, 27.0 mmol, 10 *eq*) was added into a solution of compound **3-2** (1 g, 2.7 mmol, 1 *eq)* in a mixed solvent of DCM (20 mL) and MeOH (2 mL) at 25 °C, and the resulting solution was stirred at 65 °C for 16 h; the starting materials were completely reacted and a product was produced, as indicated by LCMS; the reaction solution was filtered through diatomite, and the resulting filtrate was concentrated under reduced pressure to give compound **3-3**. MS (ESI) calcd. for C₁₇H₁₃F₃N₆O: 374; found: 375 [M+H]⁺.

Step 4: hydroxylamine hydrochloride (111.4 mg, 1.60 mmol, 1.1 *eq*) and sodium acetate (131.5 mg, 1.60 mmol, 1.5 *eq*) were added into a solution of compound **3-3** (0.5 g, 1.34 mmol, 1 *eq*) in methanol (50 mL) at 25 °C, and the resulting solution was stirred at 25 °C for 0.5 h; the starting materials were completely reacted and a product was produced, as indicated by LCMS; the reaction solution was concentrated under reduced pressure, and the resulting residue was diluted with 25 mL of saturated brine, and extracted with THF (25 mL × 3); the organic phase was dried over anhydrous sodium sulfate and filtered, and the filtrate was concentrated under reduced pressure to give compound **3-4**. MS (ESI) calcd. for C₁₇H₁₄F₃N₇O: 389; found: 390 [M+H]⁺. Step 5: thiocarbonyldiimidazole (320 mg, 1.80 mmol, 2 *eq*) was added into a solution of compound **3-4** (0.35 g, 0.89 mmol, 1 *eq*) in THF (15 mL) at 25 °C, and the resulting solution was stirred at 25 °C for 16 h; the starting materials were completely reacted and a product was produced, as indicated by LCMS; water (50 mL) was added into the reaction solution, and the aqueous phase was extracted with dichloromethane (100 mL × 3); the organic phases were pooled, dried over anhydrous sodium sulfate, and filtered; the filtrate was concentrated under reduced pressure, and the resulting residue was purified by column chromatography (SiO₂, DCM : MeOH = 50:0 to 5:1) to give compound **3-5.** MS (ESI) calcd. for C₁₇H₁₂F₃N₇O: 371; found: 372 [M+H]⁺. ¹H NMR (400 MHz, DMSO-*d*₆) δ = 12.34 (s, 1H), 8.83 (s, 1H), 7.59 - 7.58 (m, 1H), 6.96 - 6.93 (s, 1H), 6.32 (s, 1H), 5.52 (s, 1H), 4.87 - 4.79 (m, 2H), 3.14 - 3.02 (m, 2H), 2.73 - 2.68 (m, 1H), 2.34 - 2.33 (m, 1H); MS (ESI) calcd. for C₁₇H₁₂F₃N₇: 371; found: 372 [M + H]⁺.

Step 6: compound **3-5** was subjected to chiral resolution (chiral column: Chiralcel OJ-3 50 × 4.6 mm I.D., 3 µm ; mobile phases: supercritical CO₂ as phase A, and MEOH (0.05% DEA) as phase B; gradient: B content in A from 5% to 40%; flow rate: 3 mL/min; wavelength: 220 nm; column temperature: 35 °C; back pressure: 100 Bar) to give compound **3A** (retention time: 1.166 min) and compound **3B** (retention time: 1.339 min).

### Example 4: Synthesis of Compound 4

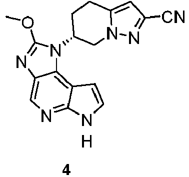

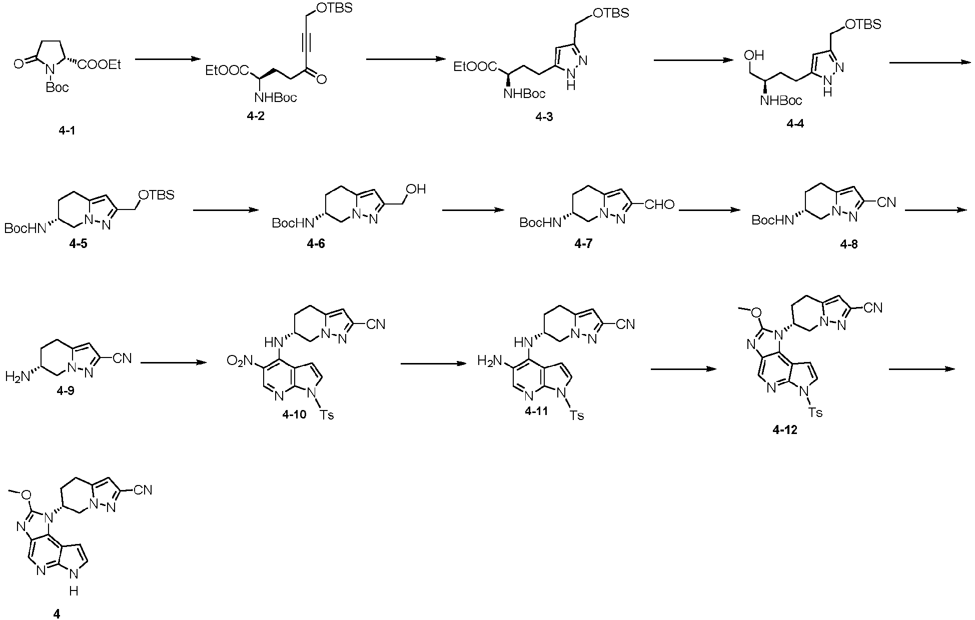

Step 1: a solution of *n*-butyllithium in *n*-hexane (2.5 M, 427.54 mL) was added dropwise into a solution of *tert*-butyldimethyl(2-propynyloxy)silane (200 g, 1174.24 mmol) in tetrahydrofuran (2 L) at -78 °C under nitrogen atmosphere, and the reaction solution was stirred at -78 °C for 30 min; a solution of compound **4-1** (250 g, 971.7 mmol) in tetrahydrofuran (2 L) was then added dropwise to the reaction solution at -78 °C; the reaction solution was allowed to react at -78 °C for 3 h; the reaction was completed as indicated by TLC (PE : EA = 3:1); the reaction was quenched with saturated aqueous ammonium chloride solution (2 L) and water (1 L), and extracted with EA (2 L × 3); the reaction solutions were pooled, washed with saturated brine (2 L), dried over anhydrous sodium sulfate, and filtered; the filtrate was concentrated under reduced pressure to give compound **4-2.** ¹H NMR (400 MHz, CDCl₃) δ = 5.11 (br d, *J* = 7.3 Hz, 1H), 4.48 (s, 2H), 4.35 - 4.27 (m, 1H), 4.21 (q, *J* = 7.2 Hz, 2H), 2.80 - 2.59 (m, 2H), 2.30 - 2.13 (m, 1H), 1.98 (br dd, *J* = 6.4, 14.2 Hz, 1H), 1.55 - 1.42 (s, 9H), 1.36 - 1.27 (m, 3H), 0.93 (s, 9H), 0.19 - 0.07 (s, 6H).

Step 2: hydrazine hydrate (34.71 g, 1.03 mol, 98%) was added into a solution of compound **4-2** (400 g, 935.44 mmol) in DMF (3 L) under ice bath; the reaction was conducted at 25 °C for 2 h; the reaction was completed as indicated by LC-MS; the reaction solution was diluted with water (10 L), and extracted with EA (2 L × 2); the reaction solutions were pooled, washed with saturated brine (2 L), dried over anhydrous sodium sulfate, and filtered; the filtrate was concentrated under reduced pressure to give compound **4-3**. MS (ESI) calcd. for C₂₁H₃₉N₃O₅Si: 441; found: 442 [M+H]⁺.

Step 3: NaBH₄ (77.71 g, 2.05 mol) was added in portions to a solution of compound **4-3** (432 g, 978.18 mmol) in THF (3 L) under ice bath; methanol (0.6 L) was then slowly added dropwise; the reaction mixture was stirred at 25 °C for 12 h, and the reaction was completed as indicated by LC-MS; the reaction was quenched with saturated aqueous ammonium chloride solution (300 mL) under ice bath, and then diluted with water (2 L); the dilution was extracted with EA (2 L × 2), and the reaction solutions were pooled, washed with saturated brine (2 L), dried over anhydrous sodium sulfate, and filtered; the filtrate was concentrated under reduced pressure, and the residue was subjected to column chromatography (SiO₂, DCM : MeOH = 50:1 to 20:1) to give compound **4-4.** MS (ESI) calcd. for C₁₉H₃₇N₃O₄Si: 399; found: 400 [M+H]⁺.

Step 4: tributyl phosphine (340.24 g, 1.68 mol) was added into a solution of compound **4-4** (336 g, 840.84 mmol) in tetrahydrofuran (4 L) under ice bath; the reaction solution was stirred under ice bath for 30 min, and ADDP (424.31 g, 1.68 mol) was added to the reaction solution. The reaction solution was stirred at 20 °C for 12 h. The reaction was completed as indicated by LC-MS. The reaction solution was diluted with water (2 L), and extracted with EA (2 L × 2); the reaction solutions were pooled, washed with saturated brine (1.5 L), dried over anhydrous sodium sulfate, and filtered; the filtrate was concentrated under reduced pressure, and the residue was subjected to column chromatography (SiO₂, PE : EA = 20:1 to 2:1) to give compound **4-5.** MS (ESI) calcd. for C₁₉H₃₅N₃O₃Si: 381; found: 382 [M+H]⁺.

Step 5: TBAF (1 M, 1.02 L, 1.02 mol) was added into a solution of compound **4-5** (390 g, 1.02 mol) in tetrahydrofuran (1 L) at room temperature, and the reaction was conducted at 20 °C for 1.5 h; the reaction was completed as indicated by LC-MS; the reaction solution was diluted with water (1 L), and the dilution was adjusted to pH = 8 with saturated aqueous NaHCO3 solution and extracted with EA (1 L × 3); the reaction solutions were pooled, washed with saturated brine (1 L), dried over anhydrous sodium sulfate, and filtered; the filtrate was concentrated under reduced pressure, and the concentrate was then dissolved in ethyl acetate (1 L); HCl/EtOAc (4 M, 200 mL) was slowly added dropwise to the resulting solution, which was then stirred for 1 h, with a white solid forming; filtration was performed to give compound **4-6.** MS (ESI) calcd. for C₁₃H₂₁N₃O₃: 267; found: 268 [M+H]⁺.

Step 6: manganese dioxide (37.40 g, 430.19 mmol) was added into a solution of compound **4-6** (11.5 g, 43.02 mmol) in DCM (150 mL) and MeOH (15 mL), which was subjected to nitrogen purging three times and then stirred at 65 °C for 12 h; the reaction was completed as indicated by LC-MS; the reaction solution was filtered, and the filtrate was concentrated to give compound **4-7.** MS (ESI) calcd. for C₁₃H₁₉N₃O₃: 265; found: 266 [M+H]⁺.

Step 7: NH₃.H₂O (51.89 g, 414.61 mmol, 57.03 mL, 28% purity) and I₂ (31.57 g, 124.38 mmol) were added into a solution of compound **4-7** (11 g, 41.46 mmol) in THF (150 mL), and the reaction solution was subjected to nitrogen purging three times and then stirred at 25 °C for 12 h; the reaction was completed as indicated by LC-MS; the reaction was quenched by adding saturated aqueous sodium sulfite solution into the reaction solution, into which 20 mL of water was added for dilution, and the dilution was extracted with ethyl acetate (50 mL × 2); the organic phases were pooled, washed with saturated brine (30 mL), dried over sodium sulfate, and filtered; the filtrate was concentrated to give a crude product; the crude product was purified by column chromatography (SiO₂, PE : EA = 3:1) to give compound **4-8;** MS (ESI) calcd. for C₁₃H₁₈N₄O₂: 262; found: 263 [M+H]⁺.

Step 8: TMSI (10.91 g, 54.52 mmol, 7.42 mL) was added to a solution of compound **4-8** (11 g, 41.94 mmol) in DCM (150 mL) at 0 °C; the reaction solution was stirred at 0 °C for 1 h; the starting materials disappeared and a new spot occurred as indicated by TLC; the reaction solution was concentrated under reduced pressure to give the hydroiodide salt of compound **4-9.** MS (ESI) calcd. for C₈H₁₀N₄: 162; found: 163 [M+H]⁺.

Step 9: DIEA (26.73 g, 206.82 mmol, 36.0 mL) was added to a solution of compound **4-9** (12 g, 41.36 mmol, hydroiodide salt) and compound 1-11 (11.64 g, 33.09 mmol) in isopropanol (200 mL); the reaction solution was subjected to nitrogen purging three times, and then stirred at 90 °C for 12 h; The reaction was completed as indicated by LC-MS. The reaction solution was cooled, and H₂O (200 mL) was added; filtration and drying were performed to give compound **4-10.** MS (ESI) calcd. for C₂₂H₁₉N₇SO₄: 477; found: 478 [M+H]⁺.

Step 10: Fe (9.36 g, 167.54 mmol) and NH₄Cl (12.55 g, 234.56 mmol) were added into a solution of compound **4-10** (16 g, 33.51 mmol) in THF (200 mL) and H₂O (50 mL), which was subjected to nitrogen purging three times and then stirred at 100 °C for 1 h. The reaction was completed as indicated by LCMS. The reaction solution was filtered, and the filtrate was diluted with H₂O (100 mL); the dilution was then extracted with ethyl acetate (150 mL × 2). The filter cake was washed with DCM : MeOH (20:1, 100 mL × 3). The extracts and filtrate were pooled, dried over sodium sulfate, and filtered; the filtrate was concentrated to give compound **4-11.** MS (ESI) calcd. for C₂₂H₂₁N₇SO₂: 447; found: 448 [M+H]⁺.

Step 11: tetramethyl orthocarbonate (456.4 mg, 3.35 mmol) was added into a solution of compound **4-11** (150 mg, 335.19 µmol) and TsOH (5.8 mg, 33.52 µmol) in AcOH (5 mL); the reaction solution was subjected to nitrogen purging three times, and then stirred at 50 °C for 2 h; the reaction was completed as indicated by LCMS; the reaction solution was concentrated to remove the solvent, and the residue was diluted with H₂O (5 mL); the dilution was extracted with dichloromethane (5 mL × 3); the organic phases were pooled, washed with saturated brine, dried over sodium sulfate, and filtered; the filtrate was concentrated to give **4-12.** MS (ESI) calcd. for C₂₄H₂₁N₇O₃S: 487; found: 488 [M+H]⁺.

Step 12: compound **4-12** (180 mg, 369.21 µmol) was dissolved in THF (10 mL), and TBAF (1 M, 738.4 µL) was then added. The reaction solution was subjected to nitrogen purging three times, and stirred at 70 °C for 12 h. The reaction was completed as indicated by LC-MS; the reaction solution was concentrated, and the residue was then diluted with aqueous NaHCO3 solution (15 mL); the dilution was extracted with DCM (15 mL × 3), and the organic phases were pooled, dried over sodium sulfate, and filtered; the filtrate was concentrated to give a crude product. The crude product was subjected to prep-HPLC (Phenomenex Gemini NX 80 × 30 mm × µm; mobile phase: [water (10 mM NH₄HCO₃)-ACN]; B(ACN)%: 20%-50%, 9 min) to isolate compound **4.** MS (ESI) calcd. for C₁₇H₁₅N₇O: 333; found: 334 [M+H]⁺. ¹H NMR (400 MHz, DMSO-*d*₆) δ = 11.72 (br s, 1H), 8.38 (br s, 1H), 7.42 (br s, 1H), 6.85 (br s, 1H), 6.71 (br s, 1H), 5.41 (br s, 1H), 4.69 (br s, 2H), 4.09 (br s, 3H), 3.05 (br s, 2H), 2.68 - 2.55 (m, 1H), 2.28 (br s, 1H).

### Example 5: Synthesis of Compound 5

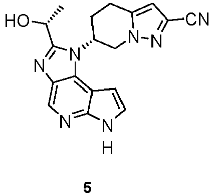

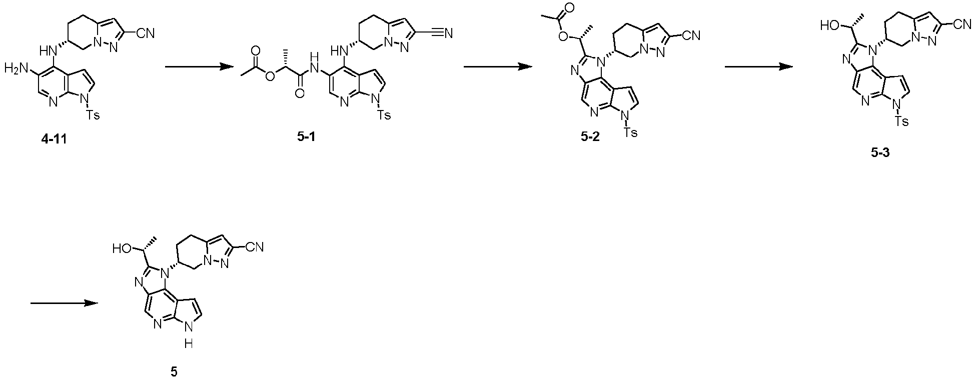

Step 1: compound **4-11** (250 mg, 558.64 µmol, 1 *eq*)*,* (2*R*)-2-hydroxypropionic acid (221.41 mg, 1.68 mmol), EDCI (321.28 mg, 1.68 mmol) and HOBt (226.46 mg, 1.68 mmol) were dissolved in THF (10 mL), and DIEA (361.00 mg, 2.79 mmol, 486.53 µL) was then added; the reaction solution was subjected to nitrogen purging three times, and then stirred at 20 °C for 12 h; the reaction was completed as indicated by LC-MS; the reaction solution was diluted with H₂O (15 mL), and the dilution was extracted with EA (15 mL × 3); the organic phases were pooled, washed with saturated brine, dried over sodium sulfate, and filtered; the filtrate was concentrated to give compound **5-1.** MS (ESI) calcd. for C₂₇H₂₇N₇O₅S: 561; found: 562 [M+H]⁺.

Step 2: compound **5-1** was dissolved in AcOH (10 mL), and stirred at 120 °C for 0.5 h; the reaction was completed as indicated by LCMS; the reaction solution was diluted with water (15 mL), and extracted with ethyl acetate (15 mL × 3); the organic phases were pooled, washed with saturated brine, dried over sodium sulfate, and filtered; the filtrate was concentrated to give compound **5-2**. MS (ESI) calcd. for C₂₇H₂₅N₇O₄S: 543; found: 544 [M+H]⁺.

Step 3: compound **5-2** (0.3 g, 551.88 µmol) was dissolved in MeOH (12 mL) and THF (3 mL), and K₂CO₃ (152.55 mg, 1.10 mmol) was then added. The reaction solution was subjected to nitrogen purging three times, and then stirred at 25 °C for 10 min. The reaction was completed as indicated by LC-MS. The reaction solution was concentrated under reduced pressure to remove the solvent, and the residue was diluted with H₂O (10 mL); the dilution was then extracted with DCM : MeOH (10:1, 10 mL × 3), and the organic phases were pooled, washed with saturated brine, dried over sodium sulfate, and filtered; the filtrate was concentrated to give compound **5-3**. MS (ESI) calcd. for C₂₅H₂₃N₇O₃S: 501; found: 502 [M+H]⁺.

Step 4: compound **5-3** (150 mg, 299.07 µmol) was dissolved in THF (5 mL), and TBAF (1 M, 598.1 µL) was then added; the reaction solution was subjected to nitrogen purging three times, and stirred at 70 °C for 12 h; the reaction was completed as indicated by LC-MS; the reaction solution was concentrated, and then diluted with aqueous NaHCO3 solution (15 mL); the dilution was extracted with DCM (15 mL × 3), and the organic phases were pooled, dried over sodium sulfate, and filtered; the filtrate was concentrated to give a crude product; the crude product was subjected to prep-HPLC (Phenomenex Gemini NX 80 × 30 mm × 3 µm ; mobile phase: [water (10 mM NH₄HCO₃)-ACN]; ACN%: 15%-45%, 9 min) to isolate and obtain compound 5. MS (ESI) calcd. for C₁₈H₁₇N₇O: 347; found: 348 [M+H]⁺. ¹H NMR (400 MHz, CD₃OD) δ = 8.64 (s, 1H), 7.44 (br d, *J* = 2.8 Hz, 1H), 6.74 (s, 1H), 6.35 (br s, 1H), 5.79 (br d, *J* = 10.4 Hz, 1H), 5.41 - 5.29 (m, 1H), 4.99 - 4.90 (m, 2H), 4.84 - 4.76 (m, 1H), 3.31 - 3.22 (m, 1H), 3.18 - 3.07 (m, 1H), 3.01 - 2.87 (m, 1H), 2.37 (br d, *J* = 12.4 Hz, 1H), 1.82 (br d, *J* = 6.3 Hz, 3H).

### Example 6: Synthesis of Compound 6

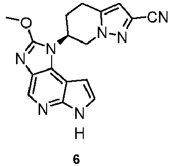

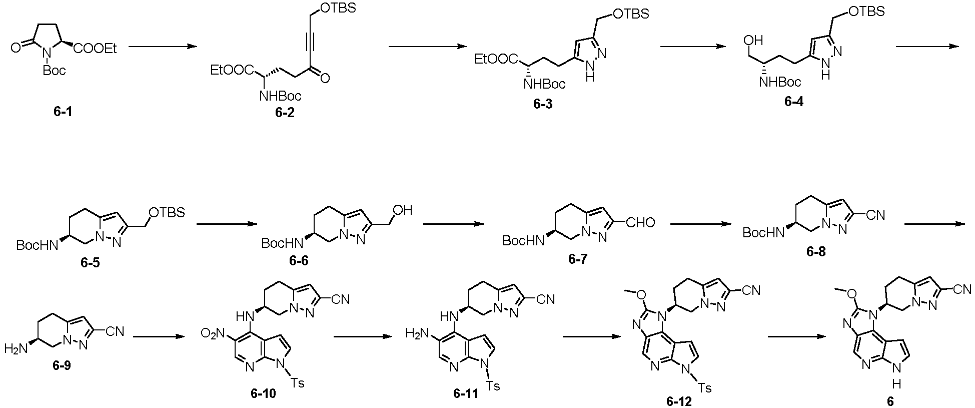

Step 1: a solution of *n*-butyllithium in *n*-hexane (2.5 M, 85.5 mL) was added dropwise into a solution of *tert*-butyldimethyl(2-propynyloxy)silane (38.07 g, 223.49 mmol, 45.3 mL) in tetrahydrofuran (500 mL) at -78 °C under nitrogen atmosphere, and the reaction solution was stirred at -78 °C for 30 min; a solution of compound 6-1 (50 g, 194.34 mmol) in tetrahydrofuran (500 mL) was then added dropwise to the reaction solution at -78 °C; the reaction solution was allowed to react at -78 °C for 3 h; the reaction was completed as indicated by TLC (PE : EA = 3:1); the reaction was quenched with saturated aqueous ammonium chloride solution (500 mL) and water (0.5 L), and extracted with EA (0.5 L × 3); the reaction solutions were pooled, washed with saturated brine (1 L), dried over anhydrous sodium sulfate, and filtered; the filtrate was concentrated under reduced pressure to give compound **6-2.**

Step 2: hydrazine hydrate (7.81 g, 231.52 mmol, 8.81 mL, 95% purity) was added into a solution of compound **6-2** (90 g, 210.47 mmol) dissolved in DMF (700 mL) at 0 °C; the reaction was conducted at 25 °C for 2 h. The reaction was completed as indicated by LC-MS; the reaction solution was diluted with water (1 L), and extracted with EA (1 L × 2); the reaction solutions were pooled, washed with H₂O (1 L × 3) and saturated brine (1 L), dried over anhydrous sodium sulfate, and filtered; the filtrate was concentrated under reduced pressure to give compound **6-3.** MS (ESI) calcd. for C₂₁H₃₉N₃O₅Si: 441; found: 442 [M+H]⁺.

Step 3: NaBH₄ (16.01 g, 423.20 mmol) was added in portions to a solution of compound **6-3** (432 g, 978.18 mmol) in THF (800 mL) at 0 °C; methanol (200 mL) was then slowly added dropwise; the reaction solution was stirred at 25 °C for 12 h, and the reaction was completed as indicated by LC-MS; the reaction was quenched with saturated aqueous ammonium chloride solution (300 mL) under ice bath, and then diluted with water (1 L); the dilution was extracted with EA (1 L × 2), and the reaction solutions were pooled, washed with saturated brine (1 L), dried over anhydrous sodium sulfate, and filtered; the filtrate was concentrated under reduced pressure, and the residue was subjected to column chromatography (SiO₂, DCM : MeOH = 50:1 to 20:1) to give compound **6-4.** MS (ESI) calcd. for C₁₉H₃₇N₃O₄Si: 399; found: 400 [M+H]⁺.

Step 4: tributyl phosphine (68.86 g, 340.34 mmol, 84.0 mL) was added into a solution of compound **6-4** (68 g, 170.17 mmol) in tetrahydrofuran (1.4 L) at 0 °C; the reaction solution was stirred at 0 °C for 30 min, and ADDP (85.87 g, 340.34 mmol) was added to the reaction solution; the reaction solution was stirred at 20 °C for 12 h; the reaction was completed as indicated by LC-MS; the reaction solution was filtered, and the filtrate was diluted with water (1 L); the dilution was extracted with EA (1 L × 2), and the reaction solutions were pooled, washed with saturated brine (1.5 L), dried over anhydrous sodium sulfate, and filtered; the filtrate was concentrated under reduced pressure, and the residue was subjected to column chromatography (SiO₂, PE : EA = 20:1 to 2:1) to give compound **6-5**. MS (ESI) calcd. for C₁₉H₃₅N₃O₃Si: 381; found: 382 [M+H]⁺.

Step 5: TBAF (1 M, 230.62 mL) was added into a solution of compound **6-5** (80 g, 209.65 mmol) dissolved in tetrahydrofuran (800 mL) at room temperature, and the reaction was conducted at 20 °C for 0.5 h; the reaction was completed as indicated by LC-MS; the reaction solution was diluted with water (1 L), and the dilution was extracted with EA (1 L × 2); the reaction solutions were pooled, washed with saturated brine (1 L), dried over anhydrous sodium sulfate, and filtered; the filtrate was concentrated under reduced pressure to give a crude product; the resulting crude product was dissolved in 800 mL of EA, and 200 mL of HCl/EA was added; the resulting mixture was stirred for 1 h to precipitate 28 g of a crude product; the crude product was dissolved in 250 mL of water, and the resulting solution was adjusted to pH 8 with sodium bicarbonate and then extracted with EA (300 mL × 2); the organic phases were pooled and concentrated to give compound 6-6. MS (ESI) calcd. for C₁₃H₂₁N₃O₃: 267; found: 268 [M+H]⁺.

Step 6: manganese dioxide (71.55 g, 822.97 mmol) was added into a solution of compound **6-6** (22 g, 82.30 mmol, 1 *eq*) in DCM (300 mL) and MeOH (30 mL), which was subjected to nitrogen purging three times and then stirred at 60 °C for 12 h; the reaction was completed as indicated by LCMS; the reaction solution was filtered, and the filtrate was concentrated to give compound **6-7**. MS (ESI) calcd. for C₁₃H₁₉N₃O₃: 265; found: 266 [M+H]⁺.

Step 7: NH₃·H₂0 (28.31 g, 226.15 mmol, 31.11 mL, 28% purity) and I₂ (17.22 g, 67.85 mmol, 13.7 mL) were added into a solution of compound **6-7** (6 g, 22.62 mmol) dissolved in THF (90 mL), and the reaction solution was subjected to nitrogen purging three times and then stirred at 20 °C for 12 h. The reaction was completed as indicated by TLC; the reaction was quenched by adding saturated aqueous sodium sulfite solution into the reaction solution and diluted with 10 mL of water, and the dilution was extracted with ethyl acetate (15 mL × 2). The organic phases were pooled, washed with saturated brine (15 mL), dried over sodium sulfate, and filtered; the filtrate was concentrated to give a crude product; the crude product was purified by column chromatography (SiO₂, PE : EA = 3:1) to isolate compound **6-8**. MS (ESI) calcd. for C₁₃H₁₈N₄O₂: 262; found: 263 [M+H]⁺.

Step 8: TMSI (9.92 g, 49.56 mmol, 6.8 mL) was added to a solution of compound **6-8** (10 g, 38.12 mmol) in DCM (100 mL) at 0 °C; the reaction solution was stirred at 0 °C for 1.5 h. The reaction was completed as indicated by LCMS. 100 mL EA was added into the reaction solution, which was then filtered to give the hydriodide salt of compound **6-9**. MS (ESI) calcd. for C₈H₁₀N₄: 162; found: 163 [M+H]⁺.

Step 9: DIEA (9.36 g, 72.39 mmol, 12.6 mL) was added to a solution of compound **6-9** (7 g, 24.13 mmol, hydroiodide salt), 4-chloro-5-nitro-1-(*p*-toluenesulfonyl)pyrrolo[2,3-*b*]pyridine (8.49 g, 24.13 mmol) dissolved in isopropanol (140 mL). The reaction solution was subjected to nitrogen purging three times, and then stirred at 90 °C for 12 h. The reaction was completed as indicated by LCMS. The reaction solution was cooled, and H₂O (100 mL) was added to precipitate a large amount of yellow solid; filtration and drying were performed to give compound **6-10**. MS (ESI) calcd. for C₂₂H₁₉N₇SO₄: 477; found: 478 [M+H]⁺.

Step 10: Fe powder (8.19 g, 146.60 mmol) and NH₄Cl (10.98 g, 205.24 mmol) were added into a solution of compound **6-10** (14 g, 29.32 mmol) in THF (168 mL) and H₂O (42 mL), which was stirred at 90 °C for 1 h. The reaction was completed as indicated by LC-MS. The reaction solution was filtered, and the filtrate was diluted with H₂O (100 mL); the dilution was then extracted with ethyl acetate (150 mL × 2); the filter cake was washed with DCM : MeOH (20:1, 100 mL × 3); the extracts and filtrate were pooled, dried over sodium sulfate, and filtered. The filtrate was concentrated to give compound **6-11**. MS (ESI) calcd. for C₂₂H₂₁N₇SO₂: 447; found: 448 [M+H]⁺.

Step 11: tetramethyl orthocarbonate (304.23 mg, 2.23 mmol) was added into a solution of compound **6-11** (100 mg, 223.46 µmol) and TsOH (3.85 mg, 22.35 µmol) dissolved in AcOH (5 mL). The reaction solution was subjected to nitrogen purging three times, and stirred at 50 °C for 12 h. The reaction was completed as indicated by LC-MS. The reaction solution was concentrated to remove the solvent, and the residue was diluted with H₂O (5 mL); the dilution was extracted with DCM (5 mL × 3). The organic phases were pooled, washed with saturated brine (5 mL), dried over sodium sulfate, and filtered; the filtrate was concentrated to give compound **6-12**. MS (ESI) calcd. for C₂₄H₂₁N₇O₃S: 487; found: 488 [M+H]⁺.

Step 12: compound **6-12** (100 mg, 205.11 µmol, 1 *eq*) was dissolved in THF (5 mL), and TBAF (1 M, 410.2 µL) was then added; the reaction solution was subjected to nitrogen purging three times, and stirred at 70 °C for 12 h. The reaction was completed as indicated by LC-MS; the reaction solution was concentrated, and the residue was then diluted with aqueous NaHCO3 solution (5 mL); the dilution was extracted with DCM (5 mL × 3), and the organic phases were pooled, dried over sodium sulfate, and filtered; the filtrate was concentrated to give a crude product. The crude product was subjected to prep-HPLC (Phenomenex Gemini NX 80 × 30 mm × 3 µm; mobile phase: [water (10 mM NH₄HCO₃)-ACN]; B(ACN)%: 20%-50%, 9 min) to isolate compound 6. MS (ESI) calcd. for C₁₇H₁₅N₇O: 333; found: 334 [M+H]⁺. ¹H NMR (400 MHz, DMSO-*d*₆) δ = 11.73 (br s, 1H), 8.38 (s, 1H), 7.43 (br s, 1H), 6.85 (s, 1H), 6.71 (br s, 1H), 5.41 (br s, 1H), 4.81 - 4.61 (m, 2H), 4.09 (s, 3H), 3.05 (br s, 2H), 2.64 - 2.54 (m, 2H).

### Example 7: Synthesis of Compound 7

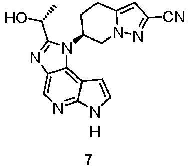

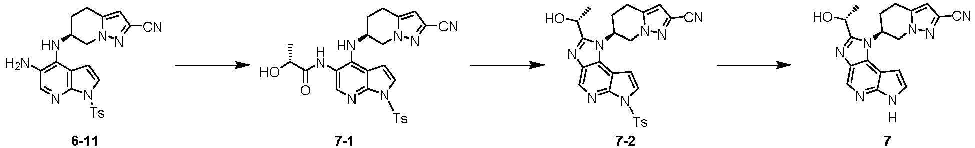

Step 1: compound 6-11 (150 mg, 335.19 µmol) and (*2R*)-2-hydroxypropionic acid (75.48 mg, 837.97 (µmol) were dissolved in THF (5 mL), and EDCI (160.64 mg, 837.97 µmol), HOBt (113.23 mg, 837.97 µmol) and DIEA (129.96 mg, 1.01 mmol, 175.2 µL) were added; the mixed solution was stirred at 20 °C for 12 h. The reaction was completed as indicated by LC-MS. H₂O (15 mL) was added into the reaction solution, which was then extracted with ethyl acetate (15 mL × 3); the organic phases were pooled, washed with saturated brine (15 mL), dried over sodium sulfate, and filtered; the filtrate was concentrated to give compound **7-1.** MS (ESI) calcd. for C₂₅H₂₅N₇O₄S: 519; found: 520 [M+H]⁺.

Step 2: compound **7-1** (200 mg, 384.93 µmol) was dissolved in AcOH (4.20 g, 69.94 mmol, 4 mL), and stirred under nitrogen atmosphere at 120 °C for 3 h. The reaction was completed as indicated by LC-MS. The reaction solution was concentrated, and the residue was diluted with aqueous NaHCO3 solution (5 mL); the dilution was extracted with DCM (5 mL × 3), and the organic phases were pooled, washed with saturated brine (15 mL), dried over sodium sulfate, and filtered; the filtrate was concentrated to give compound **7-2.** MS (ESI) calcd. for C₂₅H₂₃N₇O₃S: 501; found: 502 [M+H]⁺.

Step 3: compound **7-2** (200 mg, 398.76 µmol) was dissolved in THF (5 mL), and TBAF (1 M, 797.51 µL) was then added; the reaction solution was subjected to nitrogen purging three times, and stirred at 70 °C for 12 h. The reaction was completed as indicated by LC-MS. The reaction solution was concentrated, and the residue was then diluted with aqueous NaHCO3 solution (15 mL); the dilution was extracted with DCM (15 mL × 3), and the organic phases were pooled, dried over sodium sulfate, and filtered; the filtrate was concentrated to give a crude product. The crude product was subjected to prep-HPLC (Phenomenex Gemini NX 80 × 30 mm × 3 µm; mobile phase: [water (10 mM NH₄HCO₃)-ACN]; B(ACN)%: 17%-47%, 9 min) to isolate compound 7. MS (ESI) calcd. for C₁₈H₁₇N₇O: 347; found: 348 [M+H]⁺. ¹H NMR (400 MHz, DMSO-*d*₆) δ = 11.97 (br s, 1H), 8.62 (s, 1H), 7.60 - 7.34 (m, 1H), 6.92 (s, 1H), 6.31 (br s, 1H), 5.80 (br d, *J* = 7.0 Hz, 1H), 5.63 (br s, 1H), 5.23 (br s, 1H), 4.88 - 4.64 (m, 2H), 3.24 (br d, *J* = 12.3 Hz, 1H), 3.09 - 2.88 (m, 1H), 2.76 - 2.62 (m, 1H), 2.28 (br s, 1H), 1.67 (br d, *J* = 6.4 Hz, 3H).

### Bioactivity Assay

### Experimental Example 1: In Vitro Activity Assays of JAK1, JAK2, JAK3 and Tyk2 Kinases

### Experimental materials

Recombinant human JAK1, JAK2, JAK3 and Tyk2 proteases, and main instruments and reagents were all provided by Eurofins, UK.

### Experimental method

Dilution of JAK2, JAK3 and TYK2: 20 mM 3-(*N*-morpholino)propanesulfonic acid (MOPS), 1 mM EDTA, 0.01% Brij-35.5% glycerol, 0.1% β-mercaptoethanol, and 1 mg/mL BSA; dilution of JAK1: 20 mM TRIS, 0.2 mM EDTA, 0.1% β-mercaptoethanol, and 0.01% Brij-35.5% glycerol. All compounds were prepared as 100% DMSO solutions, and the concentration was brought to 50 times the final assay concentration. A test compound was diluted with a 3-fold gradient, with a total of 9 final concentrations ranging from 10 µM to 0.001 µM. The DMSO content in the assay reaction was 2%. The working stock solution of the compound was added into the assay wells as a first component of the reaction, and the remaining components were then added according to the protocol detailed for the assay below.

### Reaction of JAK1(h) enzyme

JAK1(h) was incubated with 20 mM Tris/HCl pH 7.5, 0.2 mM EDTA, 500 µM MGEEPLYWSFPAKKK, 10 mM magnesium acetate and [γ-³³P]-ATP (the activity and concentration were tailored as required). The reaction was started by adding a Mg/ATP mixture, and terminated by adding 0.5% phosphoric acid after 40 min of incubation at room temperature. 10 µL of the reactant was then dotted on a P30 filter pad, washed three times with 0.425% phosphoric acid and once with methanol within 4 minutes, and dried, and scintillation counting was performed.

### Reaction of JAK2(h) enzyme

JAK2(h) was incubated with 8 mM MOPS pH 7.0, 0.2 mM EDTA, 100 µM KTFCGTPEYLAPEVRREPRILSEEEQEMFRDFDYIADWC, 10 mM magnesium acetate and [γ-³³P]-ATP (the activity and concentration were tailored as required). The reaction was started by adding a Mg/ATP mixture, and terminated by adding 0.5% phosphoric acid after 40 min of incubation at room temperature. 10 µL of the reactant was then dotted on a P30 filter pad, washed three times with 0.425% phosphoric acid and once with methanol within 4 minutes, and dried, and scintillation counting was performed.

### Reaction of JAK3(h) enzyme

JAK3(h) was incubated with 8 mM MOPS pH 7.0, 0.2 mM EDTA, 500 µM GGEEEEYFELVKKKK, 10 mM magnesium acetate and [γ-³³P]-ATP (the activity and concentration were tailored as required). The reaction was started by adding a Mg/ATP mixture, and terminated by adding 0.5% phosphoric acid after 40 min of incubation at room temperature. 10 µL of the reactant was then dotted on a P30 filter pad, washed three times with 0.425% phosphoric acid and once with methanol within 4 minutes, and dried, and scintillation counting was performed.

### Reaction of TYK2(h) enzyme

TYK2(h) was incubated with 8 mM MOPS pH 7.0, 0.2 mM EDTA, 250 µM GGMEDIYFEFMGGKKK, 10 mM magnesium acetate and [γ-³³P]-ATP (the activity and concentration were tailored as required). The reaction was started by adding a Mg/ATP mixture, and terminated by adding 0.5% phosphoric acid after 40 min of incubation at room temperature. 10 µL of the reactant was then dotted on a P30 filter pad, washed three times with 0.425% phosphoric acid and once with methanol within 4 minutes, and dried, and scintillation counting was performed.

### Data analysis

The IC₅₀ results were obtained by analysis using XLFIT5 (formula 205) of IDBS company and detailed in Table 1.

**Table 1. Results of in vitro screening assays of the compounds of the present application**

| Compound | **JAK1** (IC₅₀, nM) | **JAK2** (IC₅₀, nM) | **JAK3** (IC₅₀, nM) | **TYK2** (IC₅₀, nM) |
|---|---|---|---|---|
| **1A** | 1.3 | 27.0 | 30.8 | 35.8 |
| **2A** | <1 | 2 | 25 | 2 |
| **3A** | 3 | 62 | 217 | 57 |
| **4** | 0.9 | 7.0 | 27.3 | 50.3 |
| **5** | 9 | 250 | 576 | 231 |
| **6** | 2.3 | 70.2 | 73.8 | 130 |
| **7** | 0.3 | 53.9 | 38.4 | 12.4 |

### Experimental Example 2: Pharmacokinetic (PK) Experiment

A test compound was dissolved [5% DMSO, 95% (12% SBE-β-CD)] to give a clear solution, which was administered into male rats (SD) (overnight fasting, 7-8 weeks old) by tail vein injection and by gavage, respectively. After administration of the test compound, blood was collected from the mandibular vein and centrifuged to obtain plasma at 0.117, 0.333, 1, 2, 4, 7 and 24 h in the tail vein injection group (1 mg/kg), and at 0.25, 0.5, 1, 2, 4, 8 and 24 h in the gavage group (5 mg/kg). The plasma concentration was measured using LC-MS/MS method, and the relevant pharmacokinetic parameters were calculated using WinNonlin^{™} Version 6.3 pharmacokinetic software with non-compartmental model linear logarithmic trapezoidal method. The test results were as follows:

**Table 2-1. PK test results of compounds 1A and 4 in rats**

| PK parameters | Compound 1A | Compound 4 |
|---|---|---|
| T_{1/2} (hr) | 1.35 | 3.65 |
| Cₘₐₓ (nM) | 2944 | 10152 |
| AUC_{0-inf} (nM.hr) | 8509 | 67786 |
| Bioavailability (%) | 41.8% | 119% |

| | | |
|---|---|---|
| Note: T_{1/2}: half-life; Cₘₐₓ: peak concentration; AUC_{0-inf}: area under plasma concentration-time curve from time 0 extrapolated to infinite time. | | |

### Experimental Example 3: In Vivo Efficacy Study of Adjuvant-Induced Arthritis (AIA) in Rats

Experimental procedures: the compounds of the present application were tested for their therapeutic effect on arthritis using a rat adjuvant arthritis model. Female Lewis rats weighing 160-180 g were anesthetized with isoflurane and injected with 0.1 mL of *Mycobacterium tuberculosis* suspension subcutaneously in the left hind paw. 13 days after the modeling, the rats were divided into groups and given corresponding test compounds. For example, the female Lewis rats were orally administered different doses (see Table 3-2 for specific doses) of a solution of test compound 1A in a mixed solvent [5% DMSO, 95% (12% SBE-β-CD)] twice a day (10 test animals in each dose group). During two consecutive weeks of administration, the states of the rats were observed, and the foot volume swellings were recorded and scored. The scoring criteria were shown in Table 3-1.

**Table 3-1. Clinical scoring criteria for arthritis**

| Score | Clinical symptoms |
|---|---|
| 0 | No erythema and swelling with redness |
| 1 | Erythema or mild swelling with redness near the tarsal bones or at the ankle or metatarsus, or erythema and swelling with redness at one toe |
| 2 | Mild erythema and swelling at the ankle and metatarsus, swelling with redness and erythema at two or more toes |
| 3 | Moderate erythema and swelling at the ankle, wrist, and metatarsus |
| 4 | Severe swelling with redness at all of the ankle, wrist, metatarsus and toes |

Experimental results: the two doses treatment groups of compound **1A** show significant relieving effect on the downward trend of the weight in animals caused by the disease attack, and the low and medium dose groups (3, 10 mg/kg) show significant difference from the solvent control group from day 20, showing good weight recovery effect. Compound **1A** has inhibited the increase in arthritis clinical score and foot volume, and the inhibition is dose-dependent. Compound **1A** shows the most significant effect at 10 mg/kg (significant difference from the solvent control group from day 15). The mean arthritis clinical score for this group decreases from a peak of 5.8 at day 13 to 1.3 at day 27 at the experimental endpoint.

**Table 3-2. Inhibition rate of area under clinical score curve^{∗}**

| Compound | Dose (mg/kg) | AUC (%) |
|---|---|---|
| Solvent control group | 0 | 0 |
| Compound **1A** | 3 | 49.8 |
| | 10 | 60.3 |

| | | |
|---|---|---|
| ^{∗}Note: P < 0.05 for each group compared to the solvent control group (one-way analysis of variance). | | |

### Experimental Example 4: In Vivo Efficacy Study of Collagen-Induced Arthritis (CIA) in Rats

Experimental procedures:
The compounds of the present application were tested for their therapeutic effect on arthritis using a rat collagen-induced arthritis model. Lewis rats were immunized. The day of the first immunization was recorded as day 0, and the subsequent days were recorded in order. The Lewis rats were anesthetized with isoflurane and injected with 50 µL of prepared collagen emulsion (containing 200 µg of CII) subcutaneously in the tail (2-3 cm away from the base of the tail). On day 21, the same volume of the collagen emulsion was injected in the tail in the same manner. Rats in the normal group were not immunized. On day 27 of the modeling, the rats were divided into groups and administered corresponding test compounds. For example, the female Lewis rats were orally administered different doses (see Table 4-1 for specific doses) of a solution of test compound **4** in a mixed solvent [0.5% HPMC E5/0.5% PVP K30/0.2% SLS in water] twice a day (8 test animals in each dose group). During 14 consecutive days of administration, the states of the rats were observed, and the foot volume swellings were recorded and scored. The scoring criteria were shown in Table 3-1.

Experimental results:
Compound 4 reduces the clinical scores of the arthritic rats in a dose-dependent manner when being administered BID at the doses of 1 and 3 mg/kg, and shows significant difference from the solvent control group. By the end of the administration at day 14, the 3 mg/kg BID group reduces the clinical scores of the rats to zero (Table 4-1). Meanwhile, the degree of the foot volume swelling is also reduced in a dose-dependent manner, and by the end of the administration at day 14, the 3 mg/kg BID group shows significant difference from the solvent control group in that it reduces the foot volume swelling of the rat to 1.28 µL. The body weight in the treatment group also recovers in a dose-dependent manner, and by the end of the administration at day 14, the body weight in the 3 mg/kg BID dose group recovers to the level in the normal group.

**Table 4-1. Main parameters for in vivo efficacy study of CIA in rats^{∗}**

| Compound | Dose (mg/kg) | Clinical score (on day 14 of administration) | Foot volume (µL, on day 14 of administration) |
|---|---|---|---|
| | | Mean±SEM | Mean±SEM |
| Normal group | 0 | 0 | 1.298±0.022 |
| Solvent control group | 0 | 13.20±0.82 | 2.096±0.064 |
| Dexamethasone group | 0.1 | 0.00±0.00 | 1.224±0.042 |
| Compound 4 | 1 | 0.38±0.16 | 1.324±0.040 |
| | 3 | 0.00±0.00 | 1.280±0.012 |

| | | | |
|---|---|---|---|
| ^{∗}Note: P < 0.001 for each group compared to the solvent control group (two-way analysis of variance). | | | |

## Claims

1. A compound of formula (I') or a pharmaceutically acceptable salt thereof, 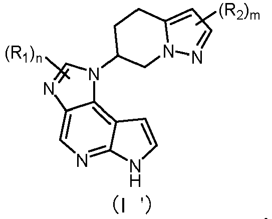 wherein,
m is 1, 2, 3, 4 or 5;
n is 1, 2, 3 or 4;
each R₁ is independently H, F, Cl, Br, I, CN, C₁₋₈ alkyl, C₁₋₈ alkoxy, C₁₋₈ alkyl S-, NH₂, C₁₋₈ alkyl NH-, (C₁₋₈ alkyl)₂N-, -COOH, or -C(O)OC₁₋₈ alkyl, wherein the C₁₋₈ alkyl, C₁₋₈ alkoxy, C₁₋₈ alkyl S-, C₁₋₈ alkyl NH-, (C₁₋₈ alkyl)₂N- or -C(O)OC₁₋₈ alkyl is each independently optionally substituted with 1, 2, 3 or 4 Rₐ;
each R₂ is independently H, F, Cl, Br, I, CN, NH₂, C₁₋₈ alkyl, C₁₋₈ alkoxy, C₁₋₈ alkyl S-, C₁₋₈ alkyl NH-, (C₁₋₈ alkyl)₂N-, or C₃₋₁₂ cycloalkyl;
each Rₐ is independently H, F, Cl, Br, I, OH, CN or NH₂.

2. The compound or the pharmaceutically acceptable salt thereof according to claim 1, wherein m is 1, 2 or 3;
alternatively, m is 1 or 2;
alternatively, m is 1.

3. The compound or the pharmaceutically acceptable salt thereof according to claim 1, wherein n is 1, 2 or 3;
alternatively, n is 1 or 2;
alternatively, n is 1;
alternatively, m and n are both 1.

4. The compound or the pharmaceutically acceptable salt thereof according to claim 1, wherein the structural fragment 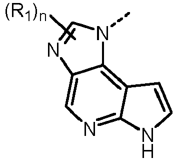 is 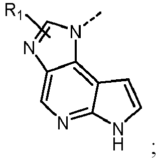 alternatively, the structural fragment

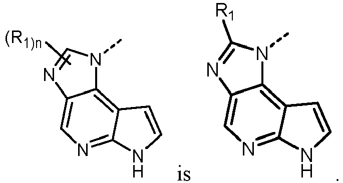

5. The compound or the pharmaceutically acceptable salt thereof according to claim 1, wherein the structural fragment 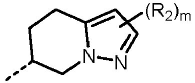 is 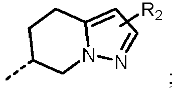 alternatively, the structural fragment 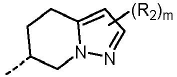 is

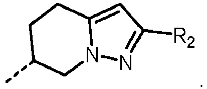

6. The compound or the pharmaceutically acceptable salt thereof according to claim 1, wherein each R₁ is independently H, F, Cl, Br, I, CN, C₁₋₆ alkyl, C₁₋₆ alkoxy, C₁₋₆ alkyl S-, NH₂, C₁₋₆ alkyl NH-, (C₁₋₆ alkyl)₂N-, -COOH, or -C(O)OC₁₋₆ alkyl, wherein the C₁₋₆ alkyl, C₁₋₆ alkoxy, C₁₋₆ alkyl S-, C₁₋₆ alkyl NH-, (C₁₋₆ alkyl)₂N- or -C(O)OC₁₋₆ alkyl is each independently optionally substituted with 1, 2, 3 or 4 Rₐ;
alternatively, wherein each R₁ is independently H, F, Cl, Br, I, CN, C₁₋₆ alkyl, C₁₋₆ alkoxy, C₁₋₆ alkyl S-, NH₂, C₁₋₆ alkyl NH-, or (C₁₋₆ alkyl)₂N-, wherein the C₁₋₆ alkyl, C₁₋₆ alkoxy, C₁₋₆ alkyl S-, C₁₋₆ alkyl NH- or (C₁₋₆ alkyl)₂N-₋ is each independently optionally substituted with 1, 2, 3 or 4 Rₐ;
alternatively, wherein each R₁ is independently H, F, Cl, Br, I, CN, C₁₋₃ alkyl, C₁₋₃ alkoxy, C₁₋₃ alkyl S-, NH₂, C₁₋₃ alkyl NH-, (C₁₋₃ alkyl)₂N-, -COOH, or -C(O)OC₁₋₃ alkyl, wherein the C₁₋₃ alkyl, C₁₋₃ alkoxy, C₁₋₃ alkyl S-, C₁₋₃ alkyl NH-, (C₁₋₃ alkyl)₂N- or -C(O)OC₁₋₃ alkyl is each independently optionally substituted with 1, 2, 3 or 4 Rₐ;
alternatively, wherein each R₁ above is independently H, F, Cl, Br, I, CN, C₁₋₃ alkyl, C₁₋₃ alkoxy, C₁₋₃ alkyl S-, NH₂, C₁₋₃ alkyl NH-, or (C₁₋₃ alkyl)₂N-, wherein the C₁₋₃ alkyl, C₁₋₃ alkoxy, C₁₋₃ alkyl S-, C₁₋₃ alkyl NH- or (C₁₋₃ alkyl)₂N- is each independently optionally substituted with 1, 2, 3 or 4 Rₐ;
alternatively, wherein each R₁ above is independently H, F, Cl, Br, I, CN, C₁₋₃ alkyl, C₁₋₃ alkoxy, C₁₋₃ alkyl S-, or C₁₋₃ alkyl NH-, wherein the C₁₋₃ alkyl, C₁₋₃ alkoxy, C₁₋₃ alkyl S- or C₁₋₃ alkyl NH- is each independently optionally substituted with 1, 2, 3 or 4 Rₐ;
alternatively, wherein each R₁ is independently H, F, Cl, Br, I, CN, C₁₋₃ alkyl, or C₁₋₃ alkoxy, wherein the C₁₋₃ alkyl or C₁₋₃ alkoxy is each independently optionally substituted with 1, 2, 3 or 4 Rₐ.

7. The compound or the pharmaceutically acceptable salt thereof according to claim 1, wherein each R₂ is independently H, F, Cl, Br, I, CN, NH₂, C₁₋₆ alkyl, C₁₋₆ alkoxy, C₁₋₆ alkyl S-, C₁₋₆ alkyl NH-, (C₁₋₆ alkyl)₂N-, or C₃₋₁₀ cycloalkyl;
alternatively, wherein each R₂ is independently H, F, Cl, Br, I, CN, NH₂, C₁₋₃ alkyl, C₁₋₃ alkoxy, C₁₋₃ alkyl S-, C₁₋₃ alkyl NH-, (C₁₋₃ alkyl)₂N-, or C₃₋₆ cycloalkyl;
alternatively, wherein each R₂ is independently H, F, Cl, Br, I, CN, NH₂, C₁₋₃ alkyl, C₁₋₃ alkoxy, C₁₋₃ alkyl S-, or C₁₋₃ alkyl NH-;
alternatively, wherein each R₂ is independently H, F, Cl, Br, I, CN or NH₂;
alternatively, wherein each R₂ is independently H, F, Cl, Br, I or CN.

8. The compound or the pharmaceutically acceptable salt thereof according to claim 1, wherein each Rₐ is independently F, Cl, Br, I or OH.

9. A compound of formula (I) or a pharmaceutically acceptable salt thereof, 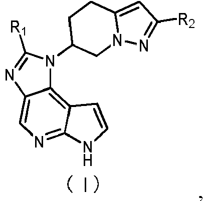 wherein,
R₁ is H, F, Cl, Br, I, CN, C₁₋₃ alkyl or C₁₋₃ alkoxy, wherein the C₁₋₃ alkyl and C₁₋₃ alkoxy are optionally substituted with 1, 2, 3 or 4 Rₐ;
R₂ is H, F, Cl, Br, I or CN;
each Rₐ is independently H, F, Cl, Br, I or OH.

10. The compound or the pharmaceutically acceptable salt thereof according to claim 1 or 9, wherein each R₁ is independently H, F, Cl, Br, I, CN, -CH₃, -CH₂CH₃, or -OCH₃, wherein the -CH₃, -CH₂CH₃ and -OCH₃ are optionally substituted with 1, 2, 3 or 4 Rₐ;
alternatively, wherein each R₁ is independently H, F, Cl, Br, I, CN, -CH₃, -CH₂CH₃ or -OCH₃, wherein the -CH₃, -CH₂CH₃ and -OCH₃ are each independently optionally substituted with 1, 2 or 3 Rₐ;
alternatively, wherein each R₁ is independently H, F, Cl, Br, I, CN, -CH₃, -CH₂CH₃, or -OCH₃, wherein the -CH₃ or -CH₂CH₃ is each independently optionally substituted with 1, 2 or 3 Rₐ;
alternatively, wherein each R₁ is independently H, F, Cl, Br, I, CN, -CH₃, -CH₂CH₃, or -OCH₃, wherein the -CH₃ or -CH₂CH₃ is each independently optionally substituted with 1, 2 or 3 F or OH;
alternatively, wherein each R₁ is independently H, F, Cl, Br, I, CN, -CH₃, -CF₃, -CH₂CH₃, -CH(OH)CH₃, or -OCH₃;
alternatively, wherein each R₁ is independently H, F, Cl, Br, I, CN, -CH₃, -CF₃, -CH₂CH₃, or -OCH₃;
alternatively, wherein each R₁ is independently H, CN, -CH₃, -CF₃, -CH(OH)CH₃, or -OCH₃;
alternatively, wherein each R₁ is independently H, CN, -CH₃, -CF₃, -CH₂CH₃, or -OCH₃.

11. The compound or the pharmaceutically acceptable salt thereof according to claim 1 or 9, wherein each Rₐ is independently F, Cl, Br, I or OH;
alternatively, wherein each Rₐ is independently F or OH.

12. The compound or the pharmaceutically acceptable salt thereof according to claim 1 or 9, wherein each R₂ is independently F, Cl, Br, I or CN;
alternatively, wherein each R₂ is independently CN.

13. The compound or the pharmaceutically acceptable salt thereof according to claim 1, which is selected from compounds of formula (I'-A) and formula (I'-B) or pharmaceutically acceptable salts thereof:

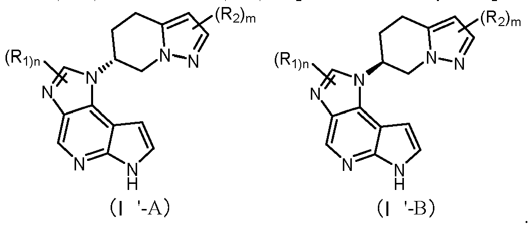

14. The compound or the pharmaceutically acceptable salt thereof according to claim 1 or 9, which is selected from compounds of formula (I-A) and formula (I-B) or pharmaceutically acceptable salts thereof:

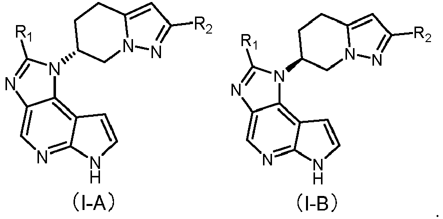

15. The compound or the pharmaceutically acceptable salt thereof according to claim 1 or 9, which has a structure of formula (I-1): 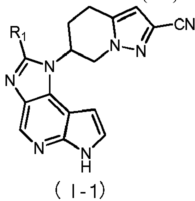 , or which is selected from formula (I-1-A) and formula (I-1-B) or pharmaceutically acceptable salts thereof:

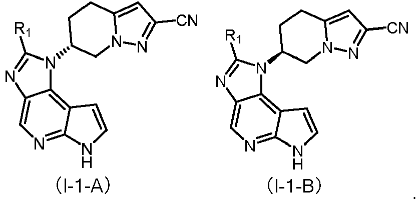

16. A compound of the formula below or a pharmaceutically acceptable salt thereof, wherein the compound is

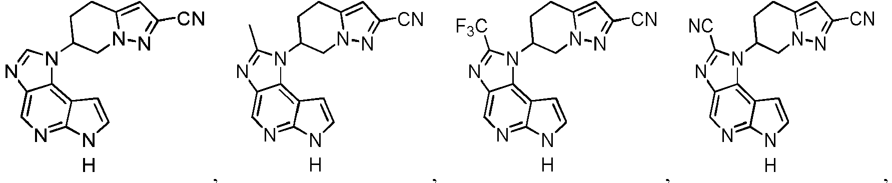

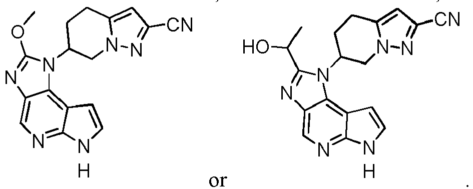

17. The compound or the pharmaceutically acceptable salt thereof according to claim 16, wherein the compound is 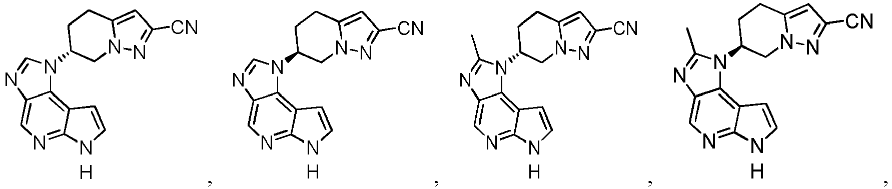 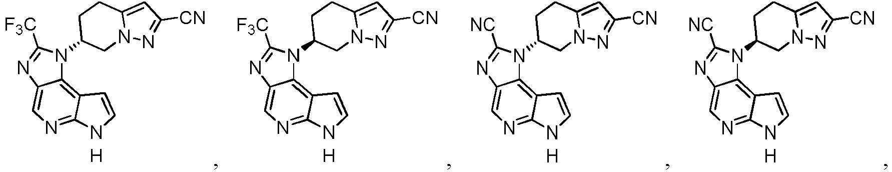 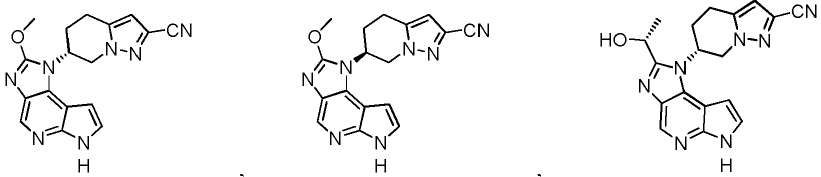 or

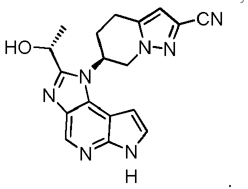

18. A pharmaceutical composition comprising the compound or the pharmaceutically acceptable salt thereof according to any one of claims 1-17.

19. Use of the compound or the pharmaceutically acceptable salt thereof according to any one of claims 1-17 or the pharmaceutical composition according to claim 18 in the preparation of a drug for treating a JAK1- and/or JAK2-associated disease, optionally, the disease is selected from inflammatory conditions.

20. A method of treating a JAK1- and/or JAK2-associated disease, comprising: administering a therapeutically effective amount of the compound or the pharmaceutically acceptable salt thereof according to any one of claims 1-17 or the pharmaceutical composition according to claim 18, optionally, the disease is selected from inflammatory conditions.
